# EUROPÄISCHE PATENTSCHRIFT

(11) **EP 1 476 537 B1**
(45) Veröffentlichungstag und Bekanntmachung des Hinweises auf die Patenterteilung: **13.07.2005**
(21) Anmeldenummer: 03742495.9
(22) Anmeldetag: 20.02.2003
(51) Int. Cl.: C12M 3/00

(54) **BEHÄLTER MIT ZUMINDEST EINER ELEKTRODE**
CONTAINER WITH AT LEAST ONE ELECTRODE
CONTENANT COMPORTANT AU MOINS UNE ELECTRODE

(30) Priorität: 20.02.2002 DE 10208188
(43) Veröffentlichungstag der Anmeldung: 17.11.2004
(73) Patentinhaber: amaxa GmbH, 50829 Köln (DE)
(72) Erfinder: MÜLLER-HARTMANN, Herbert, 50937 Köln (DE); HABIG, Michael, 50733 Köln (DE); SIEBENKOTTEN, Gregor, 50226 Frechen-Königsdorf (DE); HOFFMANN, Peter, 51063 Köln (DE)
(86) Internationale Anmeldenummer: PCT/DE2003/000536
(87) Internationale Veröffentlichungsnummer: WO 2003/070875

(56) Entgegenhaltungen:
- WO-A-01/70928
- US-A- 6 040 184
- PATENT ABSTRACTS OF JAPAN vol. 1999, no. 09, 30. Juli 1999 (1999-07-30) & JP 11 103858 A (TR TEC KK;TOKIWA SCIENCE KK), 20. April 1999 (1999-04-20)
- EUROGENTEC: "Easyject Plus User's Manual Passage" 10. Juli 1992 (1992-07-10) , EASYJECT PLUS USER'S MANUAL, XX, XX, PAGE(S) 1-27,30-39 XP002200115 Seite 12 -Seite 13

## Beschreibung

Die Erfindung betrifft einen Behälter zur Aufnahme einer wässrigen Lösung, und insbesondere von Zellen, Zellderivaten, subzellulären Partikeln und/oder Vesikeln, welcher zumindest teilweise von einer äußeren Begrenzung gebildet ist, die einen Innenraum zur Aufnahme der Lösung bildet, und welcher mindestens einen Bereich aufweist, der beim Anlegen einer elektrischen Spannung und einer anschließenden Entladung als Elektrode dient.

### Hintergrund der Erfindung

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu chemischen Methoden geringere unerwünschte Veränderungen der biologischen Struktur und Funktionen der Zielzelle bewirkt. Bei der Elektroporation werden die Fremdmoleküle aus einer wässrigen Lösung, vorzugsweise einer an die Zellen angepassten Pufferlösung oder einem Zellkulturmedium, durch einen kurzzeitigen Stromfluss, d.h. den Puls eines sich entladenden Kondensators, in die Zellen eingebracht, wobei durch die Wirkung der kurzen elektrischen Pulse die Zellmembran für die Fremdmoleküle durchlässig gemacht wird. Die Lösung bzw. Zellsuspension befindet sich dabei häufig in einer sogenannten Küvette, d.h. einem schmalen, nach oben offenen Gefäß, die in der Nähe ihres Bodens zwei gegenüberliegende, parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkem. Insbesondere beim Einbringen von DNA in tierische Zellen, der sogenannten Transfektion, entstehen aber häufig aufgrund der Labilität der Zellen besondere Probleme bei der Elektroporation, da die Überlebensrate der Zellen als wichtiger Parameter die Effizienz der Transfektion beeinflusst.

Durch das kurzzeitige Anlegen eines starken elektrischen Feldes, d.h. eines kurzen Pulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldpulses kommt es dann zur Interaktion von Membranteilen, die schließlich zur Fusion führt. Für die Elektrofusion können dabei vergleichbare apparative Vorrichtungen verwendet werden, wie für die Elektroporation.

### Stand der Technik

Behälter der eingangs genannten Art sind bekannt und werden vor allem bei der Elektroporation oder Elektrofusion in Form von Küvetten mit eingelegten Elektroden aus Metall eingesetzt. Die für diesen Zweck verwendeten Behälter sind meist schmale, nach unten geschlossene und nach oben offene Gefäße, deren Innenraum aus jeweils zwei Paaren parallel und gegenüberliegend angeordneter Seitenwände gebildet wird. Der Innenraum dient dabei der Aufnahme der Zellsuspension, d.h. in der Regel einer wässrigen Pufferlösung oder eines Zellkulturmediums, in dem die zu behandelnden Zellen suspendiert sind. Zum Anlegen einer elektrischen Spannung weisen solche Küvetten zumeist im unteren Bereich eines Paares gegenüberliegender Seitenwände ein Elektrodenpaar auf. Bei einer elektrischen Entladung fließt zwischen den beiden Elektroden ein elektrischer Strom durch die Zellsuspension, der einen Transport der Nukleinsäuren oder anderer Moleküle in die Zellen bewirkt oder je nach den gewählten Bedingungen zur Zellfusion führt. Die Elektroden bestehen dabei zumeist aus Metall, wobei häufig Aluminium verwendet wird. Diese bekannten, handelsüblichen Küvetten haben aber den Nachteil, dass bei der elektrischen Entladung Metall-Ionen in die Pufferlösung abgegeben werden, die in geringer Konzentration zu einer unerwünschten Stimulierung der Zellen führen können und in höherer Konzentration toxisch auf die Zellen wirken. So konnte beispielsweise bei der Verwendung von Aluminium-Küvetten ein negativer Effekt durch die Freisetzung von Al³⁺-Ionen nachgewiesen werden (Loomis-Husselbee et al., Biochem J 1991, 277 (Pt 3), 883 - 885). Darüber hinaus kann es bei der Verwendung von Küvetten mit Metallelektroden zur Bildung von unerwünschten Präzipitaten kommen, die ebenfalls aufgrund der Freisetzung von Metall-Ionen aus den Elektroden gebildet werden. Dabei kann es sich möglicherweise um Metallhydroxyde oder Komplexe von Metall-Ionen mit biologischen Makromolekülen aus der Pufferlösung handeln (Stapulionis, Bioelectrochem Bioenerg 1999, 48(1), 249 - 254). Aluminium-Küvetten haben schließlich auch den Nachteil, dass der Widerstand in der Küvette während der Entladung absinkt, vermutlich weil eine Schicht aus oxidiertem Aluminium mit höherem Widerstand durch den Stromfluss von der Elektrode abgelöst wird. Küvetten mit Metallelektroden sind zudem schwierig herzustellen und daher sehr teuer.

Aus der US 6,001,617 ist eine Vorrichtung zur Kultivierung von Zellen bekannt, die gleichzeitig zur Elektroporation oder Elektrofusion von Zellen verwendet werden kann. Die Vorrichtung besteht aus einem runden Behälter, der einen optisch transparenten Boden aufweist, auf dem eine Zellschicht anhaften und wachsen kann. Der Boden des Behälters kann dabei aus einem optisch transparenten, nicht leitfähigen Material bestehen, das mit einem elektrisch leitfähigen Material beschichtet ist, oder vollständig aus einem optisch transparenten und elektrisch leitfähigen Material hergestellt sein. Der elektrisch leitfähige Boden wird über eine im Wandbereich umlaufende, bandförmige Elektrode aus Metall kontaktiert. Als Gegenelektrode ist ebenfalls ein ringförmig umlaufendes Band vorgesehen. Diese bekannte Vorrichtung hat zunächst den Nachteil, dass sie vor allem für die Elektroporation von adhärenten Zellen vorgesehen und geeignet ist. Eine Transfektion von suspendierten Zellen ist hiermit nur sehr eingeschränkt und mit geringer Effizienz möglich. Aufgrund der ringförmigen, umlaufenden Kontaktierung der Bodenelektrode und der ebenfalls im Wandbereich umlaufenden Gegenelektrode kann kein homogenes elektrisches Feld erzeugt werden, so dass keine gleichmäßige Transfektion aller Zellen möglich ist. Dieser Effekt wird noch dadurch verstärkt, dass die verwendeten intrinsisch leitenden Kunststoffe als dünne Beschichtung einen hohen Widerstand aufweisen, so dass die im Zentrum der Bodenfläche anhaftenden Zellen nur mit sehr geringer Effizienz transfiziert werden können. Aufgrund seines komplexen Aufbaus und der Tatsache, dass die verwendeten intrinsisch leitenden Kunststoffe nicht spritzfähig sind, ist der bekannte Behälter darüber hinaus auch sehr teuer in der Herstellung.

### Beschreibung der Erfindung

Der Erfindung liegt daher die Aufgabe zugrunde, einen Behälter der eingangs genannten Art zu schaffen, welcher die bestehenden Nachteile vermeidet, einfach und kostengünstig herzustellen ist und darüber hinaus eine effiziente Behandlung von Zellen, Zellderivaten, subzellulären Partikeln und/oder Vesikeln mit elektrischem Strom ermöglicht.

Erfindungsgemäß wird die Aufgabe dadurch gelöst, dass mindestens eine Elektrode aus einem leitfähigen Kunststoffmaterial besteht, das zumindest auf einem Kunststoff basiert, der mit mindestens einem leitfähigen Stoff dotiert ist, wobei die Dotierung in dem Kunststoff in einer Konzentration von insgesamt 20 - 80 Gew.-% vorliegt. Durch die Verwendung eines leitfähigen Kunststoffmaterials aus dotiertem Kunststoff wird eine Freisetzung von Metall-Ionen vermieden, so dass toxische Effekte beispielsweise auf lebende Zellen, wie beispielsweise bei der Freisetzung von Al³⁺-Ionen, vermieden werden. Hierdurch kann auch die medizinische Kompatibilität der entstehenden Produkte erhöht werden, so dass beispielsweise die mögliche Verwendung von transfizierten primären Zellen zur ex vivo ― Gentherapie positiv beeinflusst wird. Durch die Dotierung des Kunststoffs mit leitfähigen Stoffen konnte bei der Entladung ein Stromfluss zwischen den beiden Elektroden erreicht werden, der dem Stromfluss bei herkömmlichen Küvetten mit Metallelektroden entspricht. Es hat sich ferner überraschenderweise herausgestellt, dass bei den verwendeten dotierten Kunststoffen bei der Elektroporation in Bezug auf die Transfektionseffizienzen verbesserte Ergebnisse gegenüber der Verwendung von beispielsweise Küvetten mit Aluminiumelektroden erzielt werden können. Dabei ist durch die Vermeidung der toxischen Effekte durch das Freisetzen von Metall-Ionen das Verhältnis von Transfektionseffizienz zu Mortalität der Zellen deutlich verbessert. Ein weiterer Vorteil der erfindungsgemäßen Behälter liegt darin, dass sich bei der elektrischen Entladung keine Präzipitate in der Lösung bilden, die an den Zellen anhaften und bei der weiteren Untersuchung bzw. Verwendung der transfizierten Zellen störend wirken können. Ein weiterer Vorteil der erfindungsgemäßen Behälter liegt offenbar auch darin, dass die verwendeten Elektroden aus dotiertem Kunststoff weniger von der verwendeten Pufferlösung beeinflusst werden. Es hat sich nämlich herausgestellt, dass sich bei der Verwendung von Aluminium-Küvetten bei der Verwendung von phosphathaltigen Puffern ohne Chlorid im Verlauf der Entladung ein sehr hoher Widerstand an der Elektrodenoberfläche aufbaut, der bei gleichem Anfangsstrom zu einem drastischen Abfallen des Stromflusses führt. Überraschenderweise ist dies bei der Verwendung der erfindungsgemäßen Behälter bzw. Elektroden nicht der Fall. Die erfindungsgemäßen Behälter verhalten sich also nur entsprechend der Leitfähigkeit der Lösung und werden ansonsten von den verwendeten Puffern nicht nachteilig beeinflusst. Da dotierte Kunststoffe im Gegensatz zu intrinsisch leitenden Kunststoffen spritzfähig sind, können die erfindungsgemäßen Behälter im 2-Komponenten-Spritzguss in einer Form gespritzt werden, so dass sie sehr einfach und kostengünstig herzustellen sind. Die Dotierung muss dabei in dem Kunststoff in einer Konzentration zwischen insgesamt 20 und 80 Gew.-% vorliegen. Unterhalb von 20 % ist die Leitfähigkeit des Kunststoffmaterials nicht mehr ausreichend, während oberhalb von 80 % die Spritzbarkeit des Kunststoffs zu stark beeinträchtigt wird. Die Dichte des dotierten Kunststoffs kann beispielsweise zwischen 1,3 und 1,5 g/cm³, vorzugsweise bei 1,37 oder 1,45 g/cm³, liegen und eine Schmelztemperatur von 230 - 310 °C aufweisen. Der spezifische Durchgangswiderstand liegt vorzugsweise bei oder unter 2 Ohm · cm, während der spezifische Oberflächenwiderstand des dotierten Kunststoffs bei oder unter 10⁴ Ohm liegt.

Es hat sich im Hinblick auf die Leitfähigkeit des Kunststoffmaterials als besonders vorteilhaft herausgestellt, wenn die Dotierung aus Kohlefasern, Graphit, Ruß, Kohlenstoffnanotubes und/oder einem intrinsisch leitenden Kunststoff besteht.

Für die Verwendung der erfindungsgemäßen Behälter bei der Elektroporation oder Elektrofusion von Zellen, Zellderivaten, subzellulären Partikeln und/oder Vesikeln hat sich in Bezug auf die Leitfähigkeit ein Anteil der Dotierung von insgesamt 20 - 60 Gew.%, weiter bevorzugt 40 - 60 Gew.-%, besonders bevorzugt 50 - 60 Gew.-%, insbesondere 55 - 60 Gew.-%, als besonders vorteilhaft erwiesen.

Bei vielen Anwendungen, insbesondere der Transfektion von DNA in den Kern primärer Zellen, können aber besonders hohe Strome bzw. Feldstärken in der Lösung erforderlich sein, um ausreichend hohe Effizienzen zu erzielen. In diesen Fällen ist es vorteilhaft, wenn die Konzentration der Dotierung in dem Kunststoff insgesamt zwischen 40 und 80 Gew.-% liegt. Hierdurch wird eine hohe Leitfähigkeit der Elektroden gewährleistet, so dass ausreichend hohe Ströme durch die Lösung bzw. den Innenraum des Behälters fließen können. Je nach Anwendung und Art der zu behandelnden Zellen kann die Konzentration der Dotierung in vorteilhafter Ausgestaltung der Erfindung bei 50 - 80 Gew.-%, vorzugsweise 60 - 80 Gew.-%, besonders bevorzugt 70 - 80 Gew.-%, insbesondere 74 - 76 Gew.%, liegen. Dabei ist auch bei hohen Konzentrationen der Dotierung bis ca. 80 Gew.-% noch eine ausreichende Spritzfähigkeit des Materials gewährleistet. Dabei kann die Spritzfähigkeit durch die Verwendung besonderer Mischdotierungen, beispielsweise Kohlefasern und Graphit, positiv beeinflusst werden.

In Bezug auf die Spritzfähigkeit des Kunststoffmaterials hat es sich als besonders vorteilhaft herausgestellt, wenn der Kunststoff Polycarbonat, Polyetheretherketon, Polypropylen, Polyamid, Polyphenylensulfid oder ein Gemisch dieser Polymere ist oder zumindest auf einem oder mehreren dieser Polymere basiert und/oder wenn der Kunststoff ein intrinsisch leitender Kunststoff ist. Besonders vorteilhaft ist dabei die Verwendung von Polyamid 66 oder Polyamid 6.

Falls der Kunststoff seinerseits mit einem intrinsisch leitenden Kunststoff dotiert ist und/oder aus intrinsisch leitendem Kunststoff besteht, ist in vorteilhafter Ausgestaltung der Erfindung vorgesehen, dass der intrinsisch leitende Kunststoff ein Polyanilin, Polyacethylen, Poly-para-phenylen, Poly-paraphenylensulfid, Polypyrrol, Polythiophen, Polypropylen oder ähnliches ist oder zumindest auf einem oder mehreren dieser Polymere basiert.

In weiterer vorteilhafter Ausgestaltung der Erfindung ist vorgesehen, dass die äußere Begrenzung aus Kunststoff, vorzugsweise transparentem Kunststoff, gebildet ist, da somit eine einfache und kostengünstige Herstellung des gesamten Behälters im Spritzgussverfahren möglich ist. Dabei kann es vorteilhaft sein, dass die Begrenzung aus dem gleichen Kunststoff besteht, auf dem auch die zumindest eine Elektrode basiert. Bei dieser Ausgestaltung können sich Vorteile bei der Verarbeitung des Kunststoffmaterials im 2-Komponenten-Spritzguß ergeben. Hierdurch wird einerseits die Herstellung vereinfacht und andererseits eine Reduzierung der Herstellungskosten erreicht. Für besondere Anwendungen kann die äußere Begrenzung aber auch aus anderen Materialien bestehen.

In besonders vorteilhafter Ausgestaltung der Erfindung ist ferner vorgesehen, dass die zumindest eine Elektrode in die äußere Begrenzung integriert ist, so dass der Behälter in einer Form gespritzt werden kann.

In einer bevorzugten Ausführungsform der Erfindung ist vorgesehen, dass der Behälter zumindest zwei Elektroden aufweist, die aus dem gleichen Material bestehen. Bei den meisten Anwendungen werden Behälter bzw. Küvetten verwendet, die zwei gegenüberliegende, parallel angeordnete Elektroden aus identischem Material aufweisen. Diese beiden Elektroden werden auf geeignete Weise kontaktiert und so an eine an die jeweiligen Anforderungen angepasste Spannungsquelle angeschlossen. In besonderen Fällen kann es aber auch von Vorteil sein, wenn zumindest zwei Elektroden aus unterschiedlichen Materialien bestehen. Dabei kann beispielsweise die Anode oder die Katode aus dem dotierten Kunststoff bestehen, während die jeweilige Gegenelektrode aus einem anderen Material, beispielsweise Edelstahl oder einem intrinsisch leitenden Kunststoff, besteht.

Als besonders vorteilhaft für die Transfektion von lebenden Zellen haben sich die Behälter mit Elektroden aus einem leitfähigen Kunststoffmaterial gemäß einem der Ansprüche 12 bis 18 erwiesen. Diese Behälter vereinen eine hohe Leitfähigkeit mit leichter Verarbeitbarkeit des Elektrodenmaterials.

In altemativer Ausgestaltung der Erfindung ist ferner vorgesehen, dass die äußere Begrenzung zumindest eine Öffnung zum Zuleiten der Lösung und zumindest eine Öffnung zum Ableiten der Lösung aufweist. Hierdurch kann der erfindungsgemäße Behälter auch als Durchflussbehälter verwendet werden, bei dem die Lösung kontinuierlich oder diskontinuierlich durch den Innenraum strömt.

Die erfindungsgemäßen Behälter eignen sich in besonders vorteilhafter Weise auch zur Verwendung in Form von Behälteranordnungen bestehend aus wenigstens 2, vorzugsweise 6, 12, 24, 48, 96 oder mehr, zu einer Einheit verbundenen Behältern, d.h. sogenannten "multi-wells".

Ein besonderer Vorteil der Erfindung liegt darin, dass die erfindungsgemäßen Behälter oder Behälteranordnungen in einem Verfahren hergestellt werden können, bei dem der Behälter oder die Behälteranordnung im 2-Komponenten-Spritzguß hergestellt werden kann, wobei zunächst die äußere Begrenzung mit mindestens einem ausgesparten Fenster gespritzt wird und anschließend zumindest in das mindestens eine Fenster das leitfähige Kunststoffmaterial aus dotiertem Kunststoff eingespritzt wird, oder wobei zunächst die mindestens eine Elektrode aus dem dotierten Kunststoff gespritzt wird und anschließend um die mindestens eine Elektrode herum die äußere Begrenzung gespritzt wird. Im Gegensatz zur Herstellung von Behältern bzw. Küvetten mit Metall-Elektroden, bei denen die Elektroden vor oder nach dem Spritzen der Behälter manuell oder maschinell in die gespritzten Rahmen bzw. Formen eingelegt werden müssen, ist erfindungsgemäß ein sehr einfaches und kostengünstiges Herstellungsverfahren möglich. Die erfindungsgemäßen Behälter oder Behälteranordnungen können in einer Form in zwei Schritten gespritzt werden, so dass sie hinsichtlich der Herstellungskosten deutlich unterhalb der herkömmlichen Kosten für Küvetten für die Elektroporation oder Elektrofusion liegen.

Die erfindungsgemäßen Behälter oder Behälteranordnungen eignen sich in besonders vorteilhafter Weise für die Verwendung in Verfahren zur Behandlung von Zellen, Zellderivaten, subzellulären Partikeln und/oder Vesikeln mit elektrischem Strom, insbesondere zur Elektroporation oder Elektrofusion, bei denen die Zellen, Zellderivate, subzellulären Partikel und/oder Vesikel zunächst in den Innenraum zumindest eines Behälters oder zumindest eines Behälters einer Behälteranordnung überführt werden, wobei der Behälter mindestens eine Elektrode aus dem dotierten Kunststoff aufweist, sowie zumindest eine weitere Elektrode vorgesehen ist, und dann eine elektrische Spannung an die Elektroden angelegt und ein Stromfluss im Innenraum des Behälters erzeugt wird. Dabei kann der elektrische Strom eine Stromdichte von bis zu 120 A/cm², vorzugsweise 80 A/cm², erreichen. Die Zellen, Zellderivate, subzellulären Partikel und/oder Vesikel können dabei in suspendierter Form, adhärent oder in sonstiger immobilisierter Form eingesetzt werden.

Die erfindungsgemäßen Behälter oder Behälteranordnungen eignen sich also neben anderen denkbaren Anwendungen beispielsweise für die Elektroporation, d.h. Verfahren zum Einbringen von biologisch aktiven Molekülen in lebende Zellen mittels elektrischen Stroms, wobei biologisch aktive Moleküle, insbesondere Nukleinsäuren, in der Lösung gelöst sind und beispielsweise durch einen Spannungspuls mit einer Feldstärke von 2 bis 10 kV*cm⁻¹ und einer Dauer von 10 bis 200 µs ein Einbringen dieser biologisch aktiven Moleküle in die Zellen erreicht wird. Auf diesen Spannungspuls kann bei besonderen Anwendungen beispielsweise ein ohne Unterbrechung folgender Stromfluss mit einer Stromdichte von 2 bis 14 A/cm², vorzugsweise 5 A/cm², und einer Dauer von 1 - 100 ms, vorzugsweise 50 ms, folgen. Die Zellen können dabei beispielsweise in Suspension oder in Form einer adhärenten Zellschicht verwendet werden.

Die erfindungsgemäßen Behälter oder Behälteranordnungen eignen sich darüber hinaus beispielsweise auch für die Elektrofusion, d.h. Verfahren zur Fusion von Zellen, Zellderivaten, subzellulären Partikeln und/oder Vesikeln mittels elektrischen Stroms, bei dem beispielsweise die Zellen, Zellderivate, subzellulären Partikel und/oder Vesikel zunächst in zweckmäßiger Dichte in einer wässrigen Lösung suspendiert werden, die Suspension anschließend in zumindest einen Behälter oder eine Behälteranordnung gemäß der vorliegenden Erfindung überführt wird, und schließlich eine elektrische Spannung an die Elektroden angelegt und ein Stromfluss durch die Suspension erzeugt wird. Alternativ können beispielsweise auch adhärente Zellen, Zellderivate, subzelluläre Partikel und/oder Vesikel oder aber auch beispielsweise adhärente Zellen mit suspendierten Zellen, Zellderivaten, subzellulären Partikeln oder Vesikeln fusioniert werden.

Die Erfindung wird im weiteren anhand der Figuren näher erläutert.

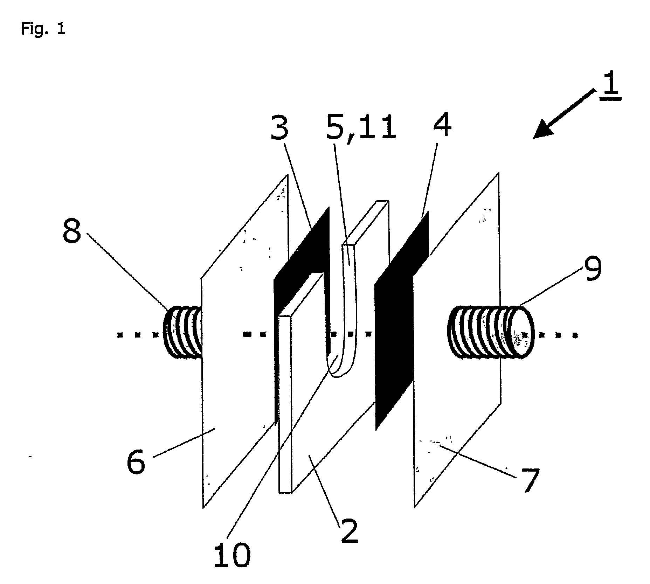

Es zeigt
- Figur 1: eine perspektivische Darstellung einer Versuchsanordnung zur Demonstration der vorliegenden Erfindung,

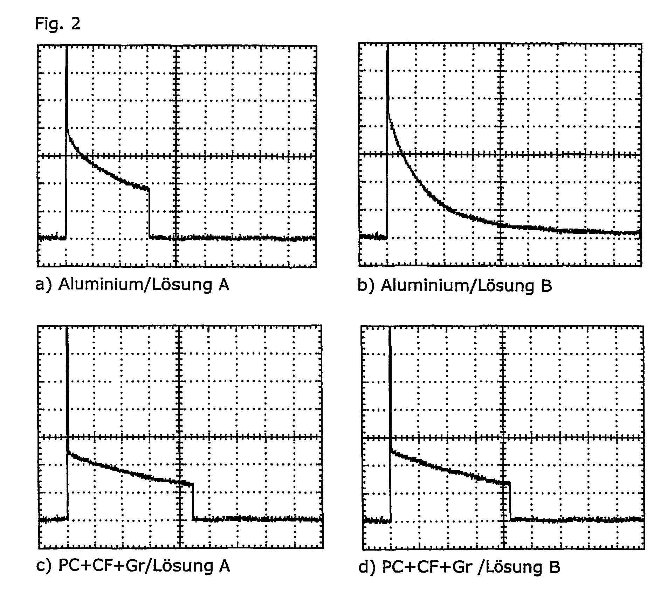

- Figur 2: Stromverläufe von elektrischen Entladungen bei Verwendung von erfindungsgemäßen Elektroden aus Polycarbonat mit 20 % Kohlefasern und 15 % Graphit (PC+CF+Gr mit einer Elektrodendicke von 1,65 mm) im Vergleich zur Verwendung von Aluminium-Elektroden jeweils mit zwei unterschiedlichen Pufferlösungen (Lösung A: 100 mM Natriumphosphat, pH 7,1 und 25 mM Kaliumchlorid; Lösung B: 140 mM Natriumphosphat, pH 7,1; Ordinate: Strom, 1 A pro Kästchen; Abszisse: Zeit, 10 ms pro Kästchen),

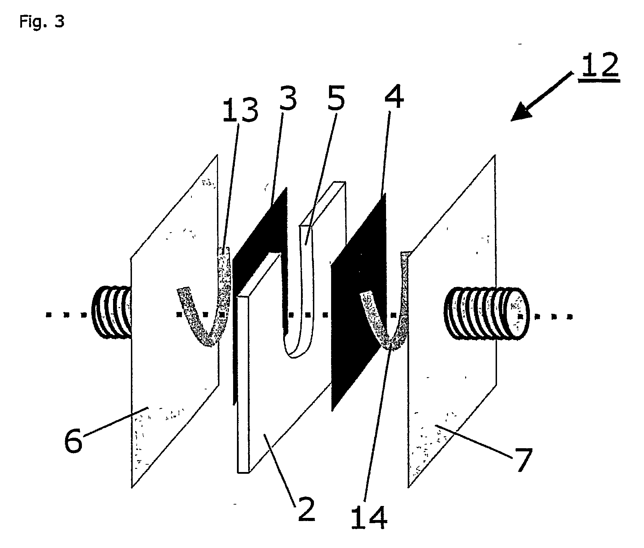

- Figur 3: eine perspektivische Darstellung einer gegenüber Figur 1 abgewandelten Versuchsanordnung mit eingelegten Kupferdrähten,

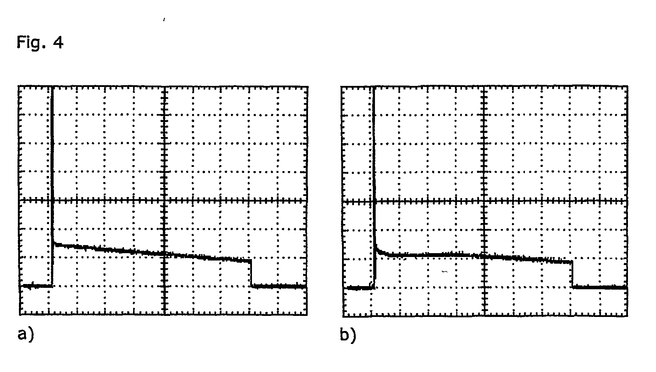

- Figur 4: Stromverläufe von elektrischen Entladungen bei Verwendung von Elektroden aus Polycarbonat mit 20 % Kohlefasern (Elektrodendicke 2 mm) für eine Versuchsanordnung gemäß Figur 1 (a) und Figur 3 (b), Ordinate: Strom, 1 A pro Kästchen und Abszisse: Zeit, 10 ms pro Kästchen,

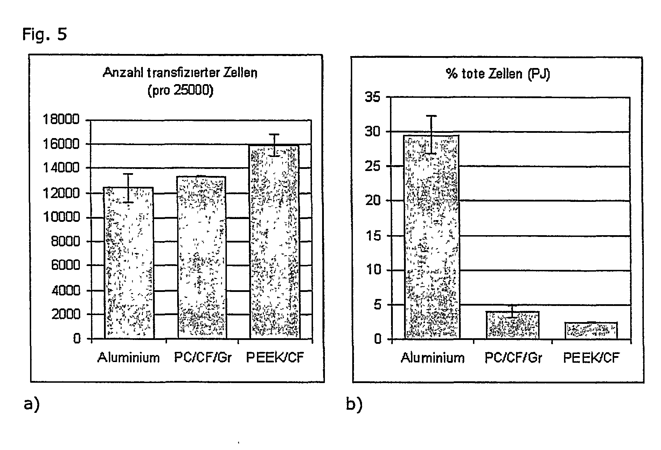

- Figur 5: Diagramme einer durchfluss-zytometrischen Analyse transfizierter CHO-Zellen (Chinesische Hamster-Ovarzellen) bei Verwendung unterschiedlicher erfindungsgemäßer Kunststoff-Elektroden im Vergleich zu Aluminium-Elektroden, a) Anzahl transfizierter Zellen pro 25000 Zellen, b) Prozentualer Anteil von mit Propidiumjodid angefärbten toten Zellen, PC/CF/Gr = Polycarbonat + 20 % Kohlefasern + 15 % Graphit mit einer Elektrodendicke von 1,65 mm, PEEK/CF = Polyetheretherketon + 40 % Kohlefasern mit einer Elektrodendicke von 1 mm,

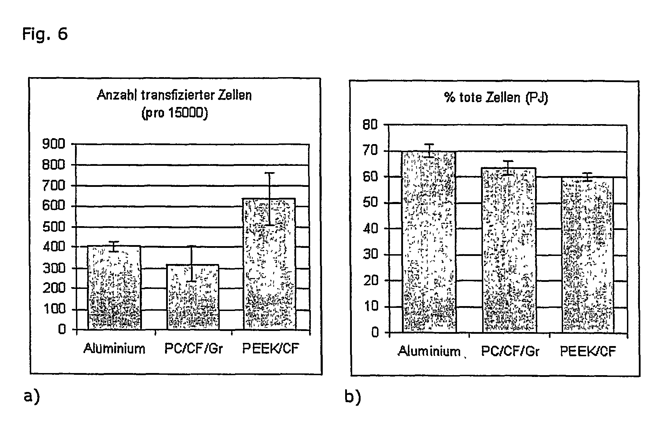

- Figur 6: Diagramme einer durchfluss-zytometrischen Analyse transfizierter HL-60-Zellen (Humane Lymphomzellen) bei Verwendung unterschiedlicher erfindungsgemäßer Kunststoff-Elektroden im Vergleich zu Aluminium-Elektroden, a) Anzahl transfizierter Zellen pro 15000 Zellen, b) Prozentualer Anteil von mit Propidiumjodid angefärbten toten Zellen, PC/CF/Gr = Polycarbonat + 20 % Kohlefasern + 15 % Graphit mit einer Elektrodendicke von 1,65 mm, PEEK/CF = Polyetheretherketon + 40 % Kohlefasern mit einer Elektrodendicke von 1 mm,

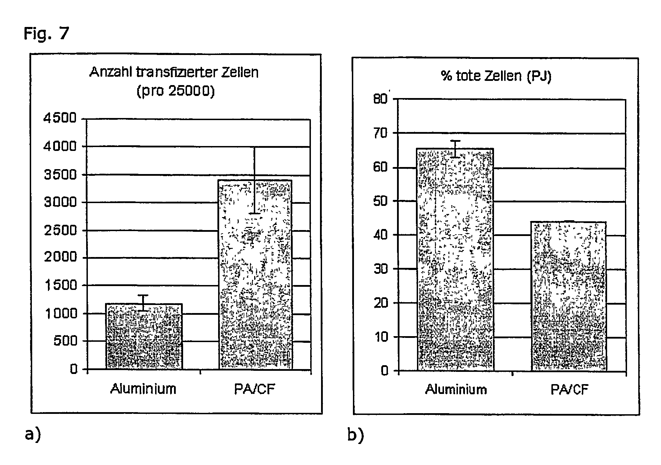

- Figur 7: Diagramme einer durchfluss-zytometrischen Analyse transfizierter Jurkat-Zellen (Humane T-Zelllinie) bei Verwendung erfindungsgemäßer Kunststoff-Elektroden im Vergleich zu Aluminium-Elektroden, a) Anzahl transfizierter Zellen pro 25000 Zellen, b) Prozentualer Anteil von mit Propidiumjodid angefärbten toten Zellen, PA/CF = Polyamid 66 + 30 % Kohlefasern mit einer Elektrodendicke von 1 mm,

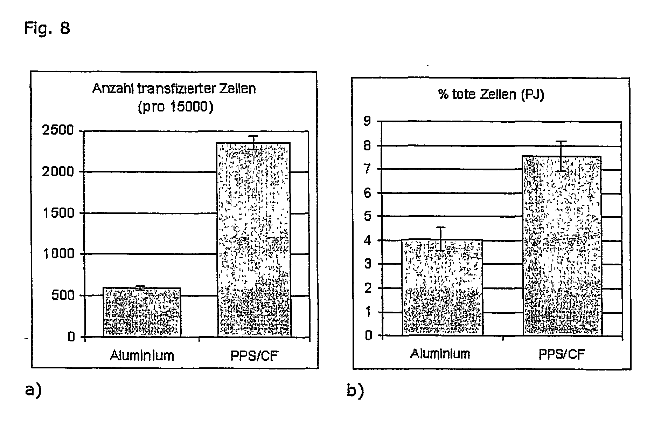

- Figur 8: Diagramme einer durchfluss-zytometrischen Analyse transfizierter HUVEC-Zellen (Humane Nabelschnurvenen-Endothelzellen) bei Verwendung erfindungsgemäßer Kunststoff-Elektroden im Vergleich zu Aluminium-Elektroden, a) Anzahl transfizierter Zellen pro 15000 Zellen, b) Prozentualer Anteil von mit Propidiumjodid angefärbten toten Zellen, PPS/CF = Polyphenylensulfid + 40 % Kohlefasern,

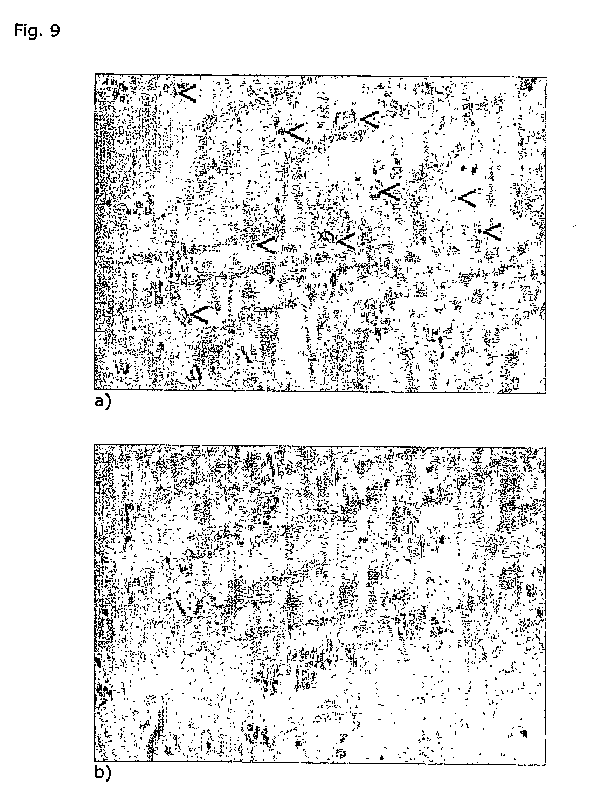

- Figur 9: mikroskopische Aufnahmen von CHO-Zellkulturen (CHO = Chinesische Hamster-Ovarzellen) 2 Tage nach einer Elektroporation mit a) Aluminium-Elektroden und b) Elektroden aus Polyphenylensulfid + 40 % Kohlefasern,

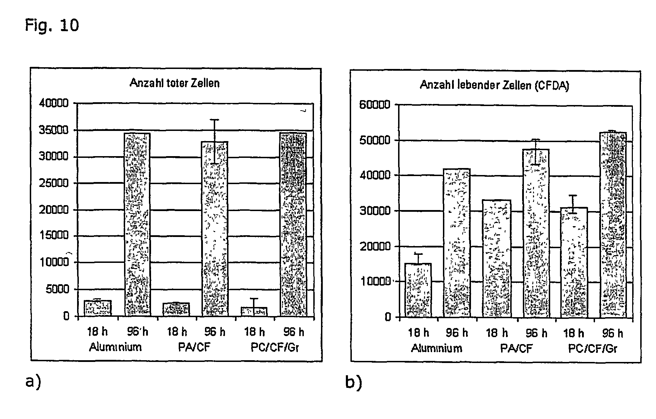

- Figur 10: Diagramme einer durchfluss-zytometrischen Analyse von HUVEC-Zellen (Humane Nabelschnurvenen-Endothelzellen) nach Inkubation in unterschiedlich behandelten Lösungen, a) Anzahl der mit Propidiumjodid gefärbten toten Zellen, b) Anzahl der mit Carboxyfluoresceindiacetat-Succinimidylester (CFDA-SE) gefärbten lebenden Zellen, PA/CF = Polyamid 66 + 30 % Kohlefasern mit einer Elektrodendicke von 1 mm, PC/CF/Gr = Polycarbonat + 20 % Kohlefasern + 15 % Graphit mit einer Elektrodendicke von 1,65 mm,

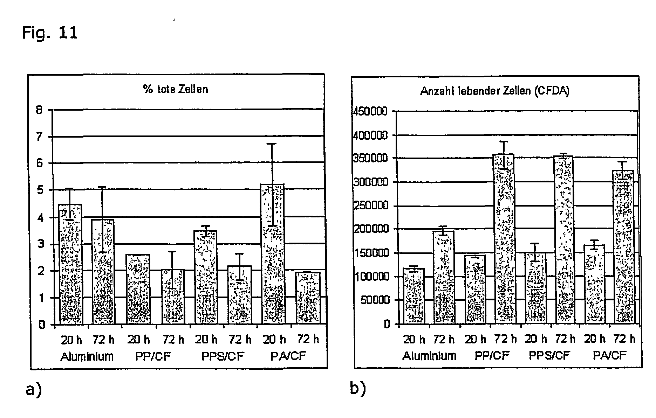

- Figur 11: Diagramme einer durchfluss-zytometrischen Analyse von CHO-Zellen (Chinesische Hamster-Ovarzellen) nach Inkubation in unterschiedlich behandelten Lösungen, a) Anzahl der mit Propidiumjodid gefärbten toten Zellen, b) Anzahl der mit Carboxyfluoresceindiacetat-Succinimidylester (CFDA-SE) gefärbten lebenden Zellen, PP/CF = Polypropylen + 20 % Kohlefasern mit einer Elektrodendicke von 1 mm, PPS/CF = Polyphenylensulfid + 40 % Kohlefasern, PA/CF = Polyamid 66 + 30 % Kohlefasern mit einer Elektrodendicke von 1 mm,

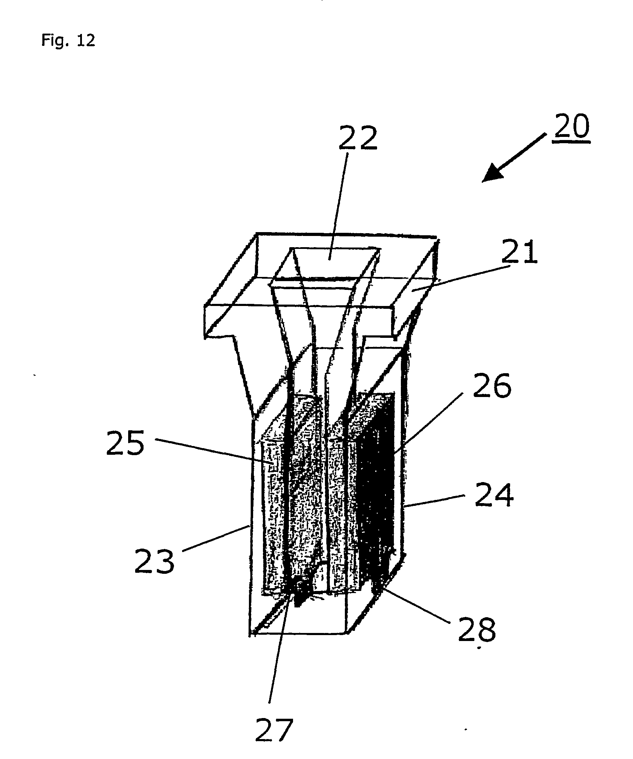

- Figur 12: eine perspektivische Darstellung einer möglichen Ausführungsform eines erfindungsgemäßen Behälters,

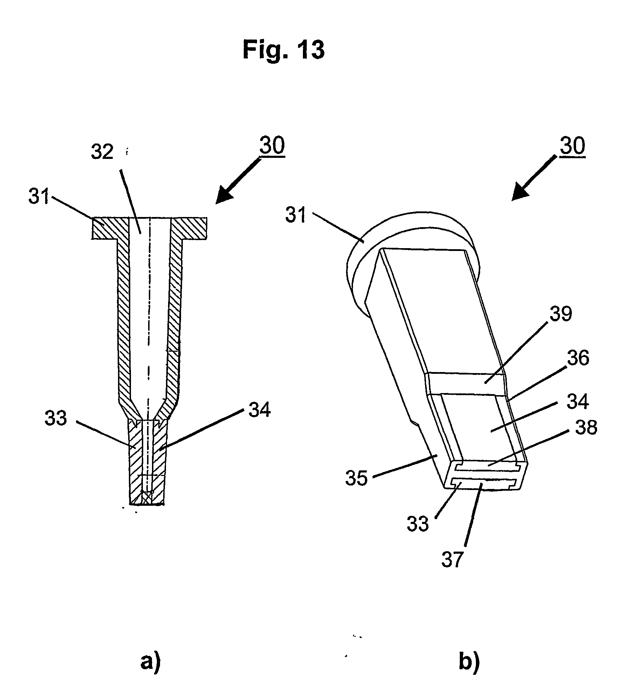

- Figur 13: eine geschnittene (a) und eine perspektivische (b) Darstellung einer weiteren Ausführungsform eines erfindungsgemäßen Behälters in Form einer Küvette,

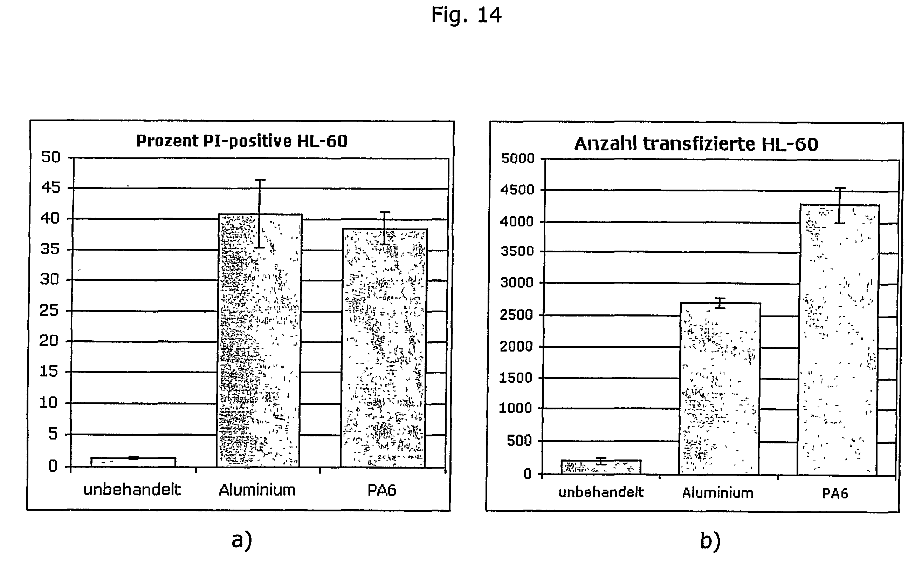

- Figur 14: Diagramme einer durchfluss-zytometrischen Analyse von HL-60-Zellen (Humane Lymphomzellen) nach Elektroporation unter Verwendung erfindungsgemäßer Kunststoff-Elektroden (PA6) im Vergleich zu Aluminium-Elektroden, a) Anzahl der mit Propidiumjodid gefärbten toten Zellen, b) Anzahl der transfizierten lebenden Zellen, unbehandelt = jeweils ohne Anlegen eines Spannungspulses,

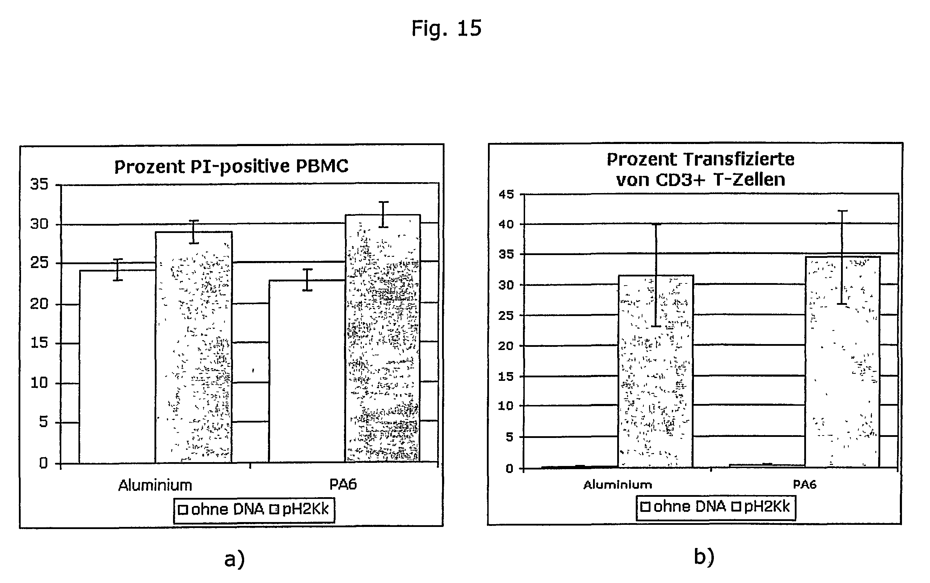

- Figur 15: Diagramme einer durchfluss-zytometrischen Analyse von CD3⁺ T-Zellen nach Elektroporation unter Verwendung erfindungsgemäßer Kunststoff-Elektroden (PA6) im Vergleich zu Aluminium-Elektroden, a) Anzahl der mit Propidiumjodid gefärbten toten Zellen, b) Anzahl der transfizierten lebenden Zellen, jeweils mit und ohne Vektor pH2-K^{k},

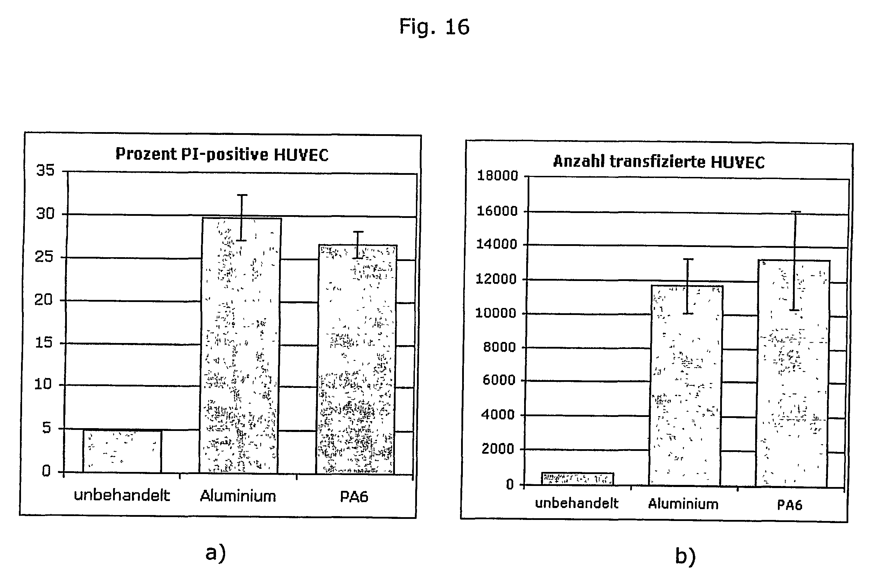

- Figur 16: Diagramme einer durchfluss-zytometrischen Analyse von HUVEC - Zellen (Humane Endothelzellen) nach Elektroporation unter Verwendung erfindungsgemäßer Kunststoff-Elektroden (PA6) im Vergleich zu Aluminium-Elektroden, a) Anzahl der mit Propidiumjodid gefärbten toten Zellen, b) Anzahl der transfizierten lebenden Zellen, unbehandelt = jeweils ohne Anlegen eines Spannungspulses, und

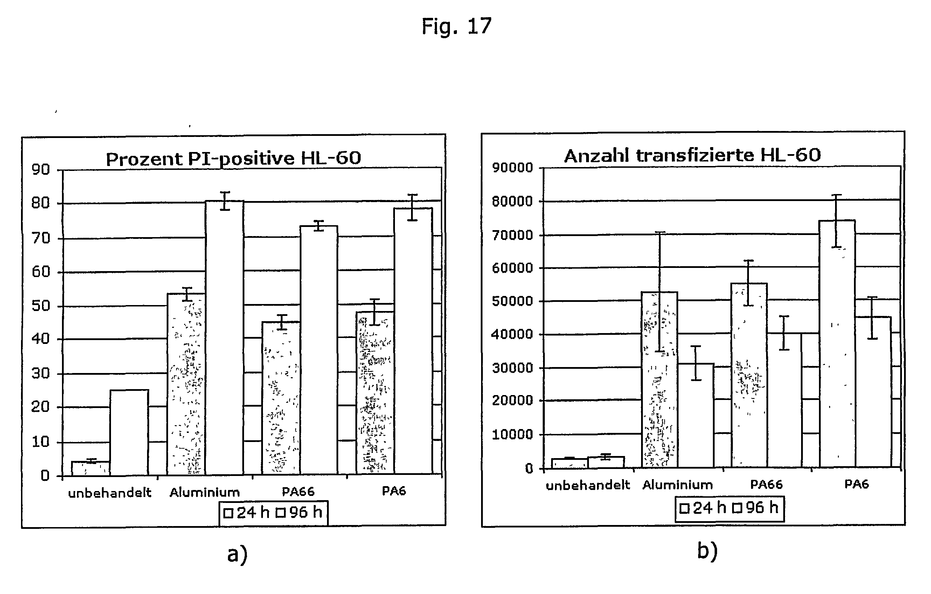

- Figur 17: Diagramme einer durchtluss-zytometrischen Analyse von HL-60-Zellen (Humane Lymphomzellen) 24 und 96 Stunden nach Elektroporation unter Verwendung erfindungsgemäßer Kunststoff-Elektroden (PA66 und PA6) im Vergleich zu Aluminium-Elektroden, a) Anzahl der mit Propidiumjodid gefärbten toten Zellen, b) Anzahl der transfizierten lebenden Zellen, unbehandelt = jeweils ohne Anlegen eines Spannungspulses.

Figur 1 zeigt eine perspektivische Darstellung einer Versuchsanordnung 1 zur Demonstration der vorliegenden Erfindung bzw. zum Testen der erfindungsgemäßen Behälter. Die Versuchsanordnung 1 entspricht dem Aufbau der erfindungsgemäßen Behälter und besteht aus einer Abstandhalterplatte 2 und zwei beidseitig an die Abstandhalterplatte 2 angepressten Elektroden 3, 4. Bei der Abstandhalterplatte 2 handelt es sich um eine 2 mm dicke Platte aus Teflon, die einen u-förmigen Ausschnitt 5 aufweist. Die Elektroden 3, 4 bestehen aus einem Kunststoff, der mit mindestens einem leitfähigen Stoff dotiert ist. Bei dem Kunststoff kann es sich beispielsweise um Polycarbonat, Polyetheretherketon, Polypropylen, Polyamid, Polyphenylensulfid oder ein Gemisch dieser Polymere und/oder einen intrinsisch leitenden Kunststoff handeln. Die 3 Schichten, bestehend aus der Abstandhalterplatte 2 und den beiden Elektroden 3, 4, werden zu beiden Seiten mittels der Kupferplatten 6, 7 sandwichartig zusammengepresst. Die Kupferplatten 6, 7 werden zu diesem Zweck mittels der Gewindeabschnitte 8, 9 einer schraubstockartigen Vorrichtung (nicht dargestellt) aufeinander zubewegt. Wenn die genannten Schichten zusammengepresst sind, entsteht im Bereich des Ausschnitts 5 ein Innenraum 10, dessen Volumen der Aufnahme der Lösung bzw. Zellen dient. Der Innenraum 10 kann im dargestellten Beispiel ein Volumen von 100 µl aufnehmen. Der Innenraum 10 wird ferner nach unten und zu zwei Seiten durch die Innenkanten 11 der Abstandhalterplatte 2 und an den beiden verbleibenden Seiten durch die Elektroden 3, 4 gebildet. Die Kupferplatten 6, 7 stehen über Drähte in elektrischen Kontakt mit den Federkontakten eines handelsüblichen Elektroporationsgerätes, d.h. einer Spannungsquelle. Die Elektroden 3, 4 stehen ihrerseits über ihre gesamte äußere Oberfläche unmittelbar mit den Kupferplatten in Kontakt, so dass eine optimale elektrische Kontaktierung gewährleistet ist. Beim Anlegen eines Spannungspulses zwischen den Kupferplatten 6, 7 fließt also bei der dargestellten Versuchsanordnung 1 ein Strom zwischen den Elektroden 3, 4 durch den mit der Lösung bzw. Zellsuspension gefüllten Innenraum 10.

Figur 2 zeigt die Stromverläufe von elektrischen Entladungen bei Verwendung von erfindungsgemäßen Elektroden aus Polycarbonat mit 20 % Kohlefasern und 15 % Graphit (PC + CF + Gr) im Vergleich zur Verwendung von Aluminium-Elektroden. Es wurde dabei der Stromfluss durch zwei unterschiedliche Pufferlösungen gemessen (Lösung A: 100 mM Natriumphosphat, pH 7,1 und 25 mM Kaliumchlorid; Lösung B: 140 mM Natriumphosphat, pH 7,1). Es wurde jeweils ein erster Spannungspuls von 1000 Volt und 40 µs Dauer gesetzt, auf den ohne Unterbrechung ein zweiter Puls mit einer Anfangsspannung (U_{Anfang}) von 90 V und einer Ladung von 75 mC folgte. Gezeigt ist hier jeweils der Stromverlauf des zweiten Pulses. Es zeigte sich dabei überraschenderweise, dass unter gleichen Bedingungen mit den erfindungsgemäßen Kunststoffelektroden größenordnungsmäßig ein ähnlicher Stromfluss erzielt werden kann, wie mit den handelsüblichen Aluminium-Elektroden (Vergleiche a und c). Die dotierten Kunststoffe eignen sich also besonders zum kurzzeitigen Leiten hoher Stromdichten. Bei der Verwendung eines Phosphatpuffers ohne Chlorid (Lösung B) zeigt sich im Gegensatz zur Verwendung eines chloridhaltigen Phosphatpuffers (Lösung A), dass sich bei Aluminium-Elektroden im Verlauf der Entladung ein sehr hoher Widerstand an der Elektrodenoberfläche aufbaut, der bei gleichem Anfangsstrom zu einem drastischem Abfall des Stromflusses führt (b). Bei der Verwendung von dotierten Kunststoffen tritt dieser negative Effekt nicht ein (d), so dass die erfindungsgemäßen Behälter bzw. Elektroden im Gegensatz zu den herkömmlichen Küvetten mit Aluminium-Elektroden auch für die Verwendung von Phosphatpuffern ohne Chlorid geeignet sind. Durch die Verwendung der erfindungsgemäßen Behälter ist man folglich in der Wahl der Pufferlösung weniger eingeschränkt als bei der Verwendung herkömmlicher Behälter.

Figur 3 zeigt eine perspektivische Darstellung einer gegenüber der in Figur 1 dargestellten Versuchsanordnung 1 abgewandelten Versuchsanordnung 12. Die Versuchsanordnung 1 gemäß Figur 1 zeigt einen Aufbau, bei dem die Elektroden 3, 4 nach außen von den Kupferplatten 6, 7 großflächig mit relativ großem Druck kontaktiert werden. Da die Kontaktierung der Elektrodenaußenseiten beispielsweise in einer herkömmlichen Elektroporationsapparatur nicht auf so großer Fläche und nicht mit so hohem Druck erfolgt, wurden bei der Versuchsanordnung 12 die Kontaktflächen zu den Elektroden kleinflächiger gestaltet. Diese Anordnung entspricht daher eher einer Kontaktierung durch Federkontakte und somit den tatsächlichen, in der Praxis vorliegenden Verhältnissen. Zu diesem Zweck wurden zwischen den Elektroden 3, 4 und den jeweils benachbarten Kupferplatten 8, 7 runde, v-förmig gebogene Kupferdrähte 13, 14 mit einem Durchmesser von ca. 1,5 mm gelegt. Figur 4 zeigt im Folgenden im Vergleich die Stromverläufe mit den Versuchanordnungen gemäß Figur 1 und 3.

Figur 4 zeigt die Stromverläufe von elektrischen Entladungen bei Verwendung von Elektroden aus Polycarbonat mit 20 % Kohlefasern jeweils für die Versuchsanordnung 1 gemäß Figur 1 (a) und die Versuchsanordnung 12 gemäß Figur 3 (b). Gezeigt sind jeweils die Stromverläufe des zweiten Pulses (1. Puls: 100 V, 40 µs; 2. Puls: U_{Anfang} = 90 V, 75 mC). Das gleichförmige Ergebnis zeigt, dass das elektrische Potential an der Elektrodeninnenseite offenbar gleichmäßig verteilt ist und die Kontaktierungsfläche folglich keinen limitierenden Faktor bezüglich der Leitfähigkeit darstellt.

Figur 5 zeigt Diagramme einer durchfluss-zytometrischen Analyse transfizierter CHO-Zellen. Um die Funktionalität der erfindungsgemäßen Behälter biologisch zu überprüfen, wurden Transfektionsversuche unter Verwendung der unter Figur 1 beschriebenen Versuchsanordnung durchgeführt. Zu diesem Zweck wurden CHO-Zellen in 100 µl einer geeigneten Pufferlösung, beispielsweise PBS (phosphat buffered saline) unter Zugabe von 5 µg des Expressions-Plasmids pH-2K^{k} (DNA-Vektor, der für die schwere Kette eines MHC Klasse I Proteins aus Maus kodiert) suspendiert und in den Innenraum der Versuchsanordnung überführt. Die Elektroporation der Zellen erfolgte dann unter Setzen von zwei Pulsen (1. Puls: 1000 V, 100 µs; 2. Puls U_{Anfang} = 108 V, 100 mC). Anschließend wurde die Zellsuspension wieder entnommen, in ein geeignetes Medium, beispielsweise RPMI-Medium, überführt und nach 20 Stunden Inkubation bei 37°C und 5 % CO₂ geemtet. Adhärente CHO-Zellen wurden mit PBS gewaschen und mit 0,1 % Trypsin + 1 mM Ethylendiamintetraacetat in PBS abgelöst. Die Expression des H-2K^{k} wurde durch Antikörperfärbung nachgewiesen (1:100 anti-H-2k^{k} von Becton Dickinson + 1:50 Beriglobin von Aventis Behring in PBS, 10 Minuten bei Raumtemperatur). Die toten Zellen wurden mit 0,25 µg/ml Propidiumjodid angefärbt. Die Analyse erfolgte in einem Durchfluss-Zytometer (FACScalibur, Becton Dickinson). Die Diagramme a) und b) zeigen, dass bei Verwendung von Elektroden mit dotiertem Kunststoff (PC/CF/Gr = Polycarbonat + 20 % Kohlefasern + 15 % Graphit und PEEK-CF = Polyetheretherketon + 40 % Kohlefasern) im Vergleich zur Verwendung von Aluminium-Elektroden zumindest vergleichbare Ergebnisse hinsichtlich der Transfektionseffizienz erzielt werden können. Aufgrund der deutlich geringeren Mortalitätsraten bei der Verwendung der erfindungsgemäßen Elektroden ergibt sich durch das günstigere Verhältnis von Transfektionseffizienz zu Mortalität sogar ein deutlicher Vorteil gegenüber der Verwendung von herkömmlichen Elektroden.

Figur 6 zeigt Diagramme einer durchfluss-zytometrischen Analyse transfizierter HL60-Zellen (Humane Lymphomzellen). Die Versuchsdurchführung und Versuchsbedingungen entsprechen den zu Figur 5 beschriebenen, mit der Ausnahme, dass die Spannungspulse bei der Elektroporation abgeändert wurden (hier: 1 Puls: 1000 V, 70 µs; 2 Puls: U_{Anfang} = 81 V, 22 mC). Auch hier konnten mit den verwendeten Elektroden aus dotiertem Kunststoff Ergebnisse erzielt werden, die denen bei Verwendung von Aluminium-Elektroden zumindest vergleichbar sind.

Figur 7 zeigt Diagramme einer durchfluss-zytometrischen Analyse transfizierter Jurkat-Zellen (Humane T-Zelllinie) bei Verwendung erfindungsgemäßer Elektroden bestehend aus Polyamid 66, welches mit 30 % Kohlefasern dotiert wurde. Die Elektroporation wurde hier in 100 µl RPMI-Medium ohne Phenolrot mit 5 µg des Plasmids pEGFP-C1 durch ein Puls von 150 Volt und 5 µs gefolgt von einem Puls mit einer Anfangsspannung von 108 V und einer Ladung von 80 mC durchgeführt. Die Analyse erfolgte hierbei nach 4 Stunden. Auch bei diesem Beispiel konnte sowohl die Transfektionseffizienz als auch die Überlebensrate durch die Verwendung der erfindungsgemäßen Behälter bzw. Elektroden aus dotiertem Kunststoff im Vergleich zu den herkömmlichen Aluminium-Elektroden deutlich erhöht werden. Die in den Figuren 5 bis 7 dargestellten Ergebnisse belegen also eindeutig, dass mit den erfindungsgemäßen Behältern die Transfektionseffizienzen bei der Elektroporation im Vergleich zu herkömmlichen Küvetten deutlich erhöht und die Mortalitätsrate deutlich gesenkt werden kann. Dies ist vor allem dadurch zu erklären, dass mit den erfindungsgemäßen Elektroden überraschenderweise vergleichbare Stromflüsse gewährleistet sind, während die bekannten Nachteile durch die Freisetzung von Metall-Ionen aus den Elektroden und somit toxische Einflüsse auf die Zellen vermieden werden.

Figur 8 zeigt Diagramme einer durchfluss-zytometrischen Analyse transfizierter HUVEC-Zellen (Humane Nabelschnurvenen-Endothelzellen) bei Verwendung von erfindungsgemäßen Elektroden aus Polyphenylensulfid mit 40 % Kohlefasern im Vergleich zu herkömmlichen Aluminium-Elektroden. Die Transfektion erfolgte in einem zellspezifischen Medium mit 5 µg/100µl Plasmid-DNA , wobei in diesem Fall zur Kompensation der geringfügig geringeren Leitfähigkeit des dotieren Kunststoffs unterschiedliche Spannungspulse gesetzt wurden (Puls für PPS/CF: 1000 V, 100 µs; Puls für Aluminium: 500 V, 100 µs). Die Zellen wurden nach 60 Stunden Inkubation durchfluss-zytometrisch auf Expression eines fluoreszierenden Proteins untersucht. Tote Zellen wurden auch hier mit 0,25 µg/ml Propidiumjodid angefärbt. Die Ergebnisse zeigen, dass die erfindungsgemäßen Behälter grundsätzlich auch für primäre menschliche Zellen geeignet sind. Dabei ist auch hier das Verhältnis von Transfektionseffizienz zu Mortalitätsrate günstiger als bei der Verwendung von herkömmlichen Aluminium-Elektroden. Dieser Effekt kann noch dadurch verstärkt werden, dass man die effektiv höheren Widerstände der dotierten Kunststoffe durch eine Erhöhung der angelegten Spannung kompensiert. Auf diese Weise kann eine Anpassung der elektrischen Verhältnisse in der Zellsuspension bei Verwendung von Elektroden aus dotiertem Kunststoff erfolgen und die Transfektionseffizienz weiter gesteigert werden.

Figur 9 zeigt mikroskopische Aufnahmen von CHO-Zellkulturen jeweils 2 Tage nach einer Elektroporation mit Aluminium-Elektroden (a) und Elektroden aus Polyphenylensulfid mit 40 % Kohlefasern (b). Auch hier wurde die geringfügig geringere Leitfähigkeit der Elektroden aus dotiertem Kunststoff durch eine Erhöhung des ersten und zweiten Spannungspulses kompensiert (PPS/CF: 1000 V, 100 µs und U_{Anfang} = 108 V, 100 mC; Aluminium: 500 V, 100 µs und U_{Anfang} = 76 V, 60 mC). Bei der Verwendung von Aluminium-Elektroden zeigen sich deutlich sichtbare Präzipitate, von denen einige in a) durch eingefügte Pfeile gekennzeichnet sind. Diese Partikel legen sich auf die Zellen. Bei einer Verwendung von Elektroden aus dotiertem Kunststoff sind solche Partikel nicht nachweisbar, so dass sich durch die Verwendung der erfindungsgemäßen Behälter ein Ausfallen von Präzipitaten offensichtlich vermeiden lässt. Dies wirkt sich zum einen positiv auf die Überlebensrate der Zellen und zum anderen vorteilhaft auf die weitere Handhabung der Zellen aus. Auch die medizinische Kompatibilität der Elektroporationsprodukte wird dadurch erhöht, so dass beispielsweise die mögliche Verwendung von transfizierten primären Zellen zur ex vivo - Gentherapie besonders vorteilhaft beeinflusst wird.

Die Figuren 10 und 11 zeigen jeweils Diagramme einer durchfluss-zytometrischen Analyse von Zellen (Figur 10: HUVEC-Zellen, Figur 11: CHO-Zellen), die jeweils in unterschiedlich behandelten Lösungen inkubiert wurden. Um die Auswirkung möglicher zellschädigender Bestandteile zu untersuchen, die aus den unterschiedlichen Elektroden aus dotiertem Kunststoff und aus Aluminium-Elektroden bei einer elektrischen Entladung frei werden könnten und über ihre unmittelbare Wirkung hinaus möglicherweise erst nach den Pulsen in der Zellkultur Wirkung zeigen, wurden die erfindungsgemäßen Behälter mit einfachen Pufferlösungen, beispielsweise PBS, ohne Zellen beladen und dreimal hintereinander einem starken Spannungspuls ausgesetzt (Aluminium: 500 V, 100 µs und U_{Anfang} = 115 V, 100 mC; dotierte Kunststoffe: 1000 V, 100 µs und U_{Anfang} = 95 V, 112 mC). Anschließend wurden 100 µl der mittels der unterschiedlichen Elektroden gepulsten Lösungen zu 400 µl Kulturmedium (EGM-2 BulletKit/Clonetics) gegeben und darin HUVEC-Zellen (18 Stunden - Werte: 5 * 10⁴ Zellen, 96 Stunden - Werte: 2,5 * 10⁴ Zellen) bzw. CHO-Zellen (20 Stunden - Werte: 10⁵ Zellen, 72 Stunden - Werte: 2* 10⁴ Zellen) in 24-wellPlatten ausgesät. Die Anzahl der toten bzw. lebenden Zellen wurde nach unterschiedlichen Zeiten und einer Inkubation bei 37°C und 5 % CO₂ durchfluss-zytometrisch ermittelt. Nach Ablösen der Zellen durch 1 µg/ml Trypsin in 1 mM Ethylendiaminthetraacetat in PBS und Vereinigung dieser Zellen mit dem Kulturüberstand wurde zur Ermittlung der toten Zellen 0,25 µl/ml Propidiumjodid zugefügt. Zur Anfärbung der lebenden Zellen wurde 0,2 µM Carboxyfluoresceindiacetat-Succinimdylester (CFDA-SE) in PBS + 0,5 % Rinderserumalbumin zugegeben, und vor der durchfluss-zytometrischen Analyse für 2 Minuten bei Raumtemperatur inkubiert. Um die absolute Anzahl von Zellen in einem Ansatz zu ermitteln, wurde eine definierte Anzahl von Flow-Count Fluorosphere Beads (Beckman Coulter) zugefügt, die sich im FACS von den Zellen unterscheiden lassen. Auf diese Weise ließ sich die ermittelte Zellzahl auf das gesamte Volumen in der Zellkulturvertiefung extrapolieren. Die in den Figuren 10 und 11 dargestellten Ergebnisse zeigen, dass sich bei der Verwendung von Elektroden aus dotiertem Kunststoff im Vergleich zu Aluminium-Elektroden ein leichter Vorteil ergibt. Insbesondere nach 4 Tagen sind die unter Verwendung von Kunststoff-Elektroden gepulsten Lösungen für das Überleben und Wachstum von CHO-Zellen günstiger. Bei HUVEC-Zellen kann man schon nach 24 Stunden einen positiven Effekt der verwendeten Kunststoffelektroden im Vergleich zu den herkömmlichen Aluminium-Elektroden erkennen. Diese Ergebnisse geben also einen Hinweis auf eine bessere Verträglichkeit von Elektroden aus dotiertem Kunststoff im Hinblick auf die Freisetzung zellschädigender Bestandteile, wobei hier die negativen Auswirkungen nach dem Stromfluss untersucht wurden. Durch die Vermeidung der Freisetzung toxischer Metall-Ionen wird zusätzlich eine bessere biologische Kompatibilität der erfindungsgemäßen Behälter erreicht. Diese sind folglich vor allem im Hinblick auf eine weitere Verwendung der transfizierten Zellen, beispielsweise eine Verwendung von veränderten primären Zellen zur ex vivo - Gentherapie, deutlich gegenüber herkömmlichen Behältern bzw. Küvetten im Vorteil.

Figur 12 zeigt eine perspektivische Darstellung einer möglichen Ausführungsform eines erfindungsgemäßen Behälters. Der hier dargestellte Behälter 20 weist im wesentlichen die Form einer herkömmlichen Küvette auf. Der Behälter wird durch eine äußere Begrenzung 21 gebildet, welche ihrerseits einen Innenraum 22 bildet, der zur Aufnahme einer wässrigen Lösung dient. In der wässrigen Lösung können beispielsweise Zellen, Zellderivate, subzelluläre Partikel und/oder Vesikel suspendiert sein. Der Behälter kann beispielsweise zusätzlich zur wässrigen Lösung oder Suspension auch adhärente Zellen, Zellderivate, subzelluläre Partikel und/oder Vesikel enthalten. In zwei parallel und gegenüberliegend angeordneten Seitenwänden 23, 24 der äußeren Begrenzung 21 finden sich zwei gleichfalls parallele Elektroden 25, 26. Die beiden Elektroden 25, 26 bestehen aus einem Kunststoffmaterial, welches erfindungsgemäß mit zumindest einem leitfähigen Stoff dotiert ist. Bei der Dotierung kann es sich beispielsweise um Kohlefasern, Graphit, Ruß, Kunststoffnanotubes oder einen intrinsisch leitenden Kunststoff, oder aber auch um eine Kombination einer oder mehrerer dieser Stoffe handeln. Die äußere Begrenzung 21 besteht aus einem transparenten Kunststoffmaterial, dass elektrisch nicht-leitend ist. Der erfindungsgemäße Behälter 20 kann aufgrund der Spritzfähigkeit aller seiner Komponenten im 2-Komponenten-Spritzguß hergestellt werden. Dabei wurde im vorliegenden Fall zunächst die äußere Begrenzung 21 aus dem nicht-leitenden Kunststoff gespritzt. In die ausgesparten Fenster (nicht mehr sichtbar) wurde durch die Spritzkanäle 27, 28 der ebenfalls spritzfähige dotierte Kunststoff eingespritzt. Auf diese Weise ist eine äußerst einfache und kostengünstige Herstellung des erfindungsgemäßen Behälters 20 möglich. Ein solcher Behälter 20 in Küvettenform ist vor allem dann vorteilhaft, wenn dieser in einer herkömmlichen Elektroporationsapparatur verwendet werden soll. Je nach Art der Anwendung sind allerdings auch alle sonstigen denkbaren und sinnvollen Formen für einen erfindungsgemäßen Behälter möglich.

Figur 13 zeigt eine perspektivische Darstellung (b) einer möglichen Ausführungsform eines erfindungsgemäßen Behälters sowie einen Längsschnitt (a) durch denselben. Der hier dargestellte Behälter 30 weist ebenfalls die Form einer Küvette auf. Der Behälter wird durch eine äußere Begrenzung 31 gebildet, welche ihrerseits einen Innenraum 32 bildet, der zur Aufnahme einer wässrigen Lösung dient In zwei parallel und gegenüberliegend angeordneten Seitenwänden 35, 36 der äußeren Begrenzung 31 sind zwei gleichfalls parallele Elektroden 33, 34 angeordnet Die beiden Elektroden 33, 34 bestehen aus einem Kunststoffmaterial, beispielsweise Polyamid 66 oder Polyamid 6, welches erfindungsgemäß mit zumindest einem leitfähigen Stoff dotiert ist Bei der Dotierung kann es sich in vorteilhafter Ausgestaltung der Erfindung beispielsweise um eine Mischung aus Kohlefasern und Graphit handeln. Die äußere Begrenzung 31 besteht aus einem transparenten Kunststoffmaterial, dass elektrisch nicht-leitend ist. Der erfindungsgemäße Behälter 30 kann aufgrund der Spritrfähigkeit aller seiner Komponenten im 2-Komponenten-Spritzguß hergestellt werden. Dabei kann zunächst die äußere Begrenzung 31 aus dem nicht-leitenden Kunststoff gespritzt, und in die ausgesparten Fenster durch die Spritzkanäle 37, 38 anschließend der ebenfalls spritzfähige dotierte Kunststoff eingespritzt werden. Auf diese Weise ist eine äußerst einfache und kostangünstige Herstellung des erfindungsgemäßen Behälters 30 möglich. Der Behälter 30 weist ferner in der unteren Hälfte eine Abschrägung 39 auf, die eine Anpassung seiner Form an die Geometrie der entsprechenden Aufnahmevorrichtung des verwendeten Gerätes, beispielsweise des Elektroporators, ermöglicht. Femer kann durch unterschiedliche Ausprägung der Abschrägung 39 der Abstand der Elektroden 33, 34 zueinander variiert und somit der Durchgangswiderstand verändert werden.

Die Figuren 14 - 16 zeigen Diagramme durchfluss-zytometrischer Untersuchungen transfizierter Zellen bei Verwendung von Küvetten mit erfindungsgemäßen Elektroden gemäß Figur 13, die einen Abstand zwischen den Elektroden von 1,5 mm aufweisen, im direkten Vergleich zu Küvetten mit Aluminiumelektroden mit einem Elektrodenabstand von 2 mm. Durch die Reduktion des Elektrodenabstands um 25% wurde der höhere Materialwiderstand des dotierten Kunststoffs im Vergleich zu Aluminium kompensiert, um die effektiv gleiche Leitfähigkeit pro Querschnittsfläche zu erreichen. Die experimentelle Vorgehensweise bei den Kunststoffküvetten entspricht der bei Aluminium, wobei ein Elektroporator mit Federmessingkontakten (Nucleofector™ I, amaxa GmbH, Köln) verwendet wurde. Als Lösungen zur Aufnahme der Zellen wurden jeweils zelltypspezifische Nucleofector™ Kits (amaxa GmbH, Köln) verwendet. Die erfindungsgemäßen Küvetten weisen jeweils Elektroden aus mit ca. 38 - 42 Gew.-% Kohlefasern und ca. 33 - 37 Gew.-% Graphit dotiertem Polyamid (PA6 oder PA66) auf (Konzentration der Dotierung insgesamt: ca. 70 - 80 Gew.-%).

Figur 14 zeigt Diagramme einer durchfluss-zytometrischen Analyse von HL-60 - Zellen (Humane Lymphomzellen) nach Elektroporation unter Verwendung erfindungsgemäßer Kunststoff-Elektroden (PA6) im Vergleich zu Aluminium-Elektroden. Je 10⁶ HL-60 Zellen (ATCC) wurden in 100 µl Lösung aufgenommen und mit je 2 µg pEGFP-C1-DNA (Clontech) versetzt. Diese Ansätze wurden in unterschiedlichen Küvetten zwei unmittelbar aufeinanderfolgenden Spannungspulsen (1000 V, 100 µs und U_{Anfang} = 90 V, 75 mC) ausgesetzt und direkt in *Iscove's modified Dulbecco's medium* mit L-Glutamin und 20% fötalem Kälberserum (Gibco) im Brutschrank bei 37°C / 5% CO2 inkubiert. Die Zellen wurden nach 24 h geerntet und mit Propidiumiodid sowie 25.000 APC-markierten Beads (Becton Dickinson) pro Ansatz versetzt. Hierdurch wurde die Bestimmung der Propidiumiodid-gefärbten Zellen und der transfizierten Zellen auf der Basis absoluter Zahlen von Zellen je Ansatz im Durchflusszytometer (FASCalibur, Becton Dickinson) möglich. Es zeigt sich hier, dass durch die Verwendung der erfindungsgemäßen Küvetten, im Vergleich zu herkömmlichen Küvetten mit Aluminiumelektroden, die Transfektionseffizienz deutlich gesteigert und die Mortalitätsrate leicht verringert werden konnte.

Figur 15 zeigt Diagramme einer durchfluss-zytometrischen Analyse von CD3⁺ T-Zellen nach Elektroporation unter Verwendung erfindungsgemäßer Kunststoff-Elektroden (PA6) im Vergleich zu Aluminium-Elektroden. Je 5x10⁶ frisch isolierte PBMC wurden in 100 µl Lösung aufgenommen und mit je 2 µg pH-2K^{k} (mouse MHC I heavy chain) versetzt. Diese Ansätze wurden in unterschiedlichen Küvetten zwei unmittelbar aufeinanderfolgenden Spannungspulsen (1000 V, 100 µs und U_{Anfang} = 96 V, 56 mC) ausgesetzt und direkt in AIM-V Medium mit 10% fötalem Kälberserum (Gibco) im Brutschrank bei 37°C / 5% CO2 inkubiert. Die Zellen wurden nach 24 h geerntet und mit Propidiumiodid sowie 25.000 APC-markierten Beads (Becton Dickinson) pro Ansatz versetzt. Zusätzlich wurden die Zellen mit einem Fluorescein-Isothiocyanat-gefärbten anti-H-2K^{k}-Antikörper (Becton Dickinson) sowie einem Antikörper gegen das menschliche T-Zell-spezifische CD3-Antigen gefärbt, das an APC gekoppelt war (Becton Dickinson). Hierdurch wurde die Bestimmung der Propidiumiodid-gefärbten Zellen und der transfizierten T-Zellen auf der Basis absoluter Zahlen von Zellen je Ansatz im Durchflusszytometer (FASCalibur, Becton Dickinson) möglich. Es konnten hier in etwa vergleichbare Ergebnisse erzielt werden.

Figur 16 zeigt Diagramme einer durchfluss-zytometrischen Analyse von menschlichen Nabelschnurvenen-Endothelzellen (HUVEC) nach Elektroporation unter Verwendung erfindungsgemäßer Kunststoff-Elektroden (PA6) im Vergleich zu Aluminium-Elektroden. Je 6,8x10⁵ HUVEC - Zellen wurden in 100 µl Lösung aufgenommen und mit je 2 µg pEGFP-C1-DNA (Clontech) versetzt. Diese Ansätze wurden in unterschiedlichen Küvetten einem Spannungspuls (1000 V, 100 µs) ausgesetzt und direkt in EGM-2 Medium für Endothelzellen (Clonetics) im Brutschrank bei 37°C / 5% CO2 inkubiert. Die Zellen wurden nach 24 h geerntet und mit Propidiumiodid sowie 25.000 APC-markierten Beads (Becton Dickinson) pro Ansatz versetzt. Hierdurch wurde die Bestimmung der Propidiumiodid-gefärbten Zellen und der transfizierten Zellen auf der Basis absoluter Zahlen von Zellen je Ansatz im Durchflusszytometer (FASCalibur, Becton Dickinson) möglich. Es zeigt sich hier, dass durch die Verwendung der erfindungsgemäßen Küvetten, im Vergleich zu herkömmlichen Küvetten mit Aluminiumelektroden, die Transfektionseffizienz gesteigert und die Mortalitätsrate verringert werden konnte.

Figur 17 zeigt Diagramme einer durchfluss-zytometrischen Analyse von HL-60 - Zellen (Humane Lymphomzellen) 24 und 96 Stunden nach Elektroporation unter Verwendung erfindungsgemäßer Kunststoff-Elektroden (PA66 und PA6) im Vergleich zu Aluminium-Elektroden. Die Bedingungen und Vorgehensweisen entsprechen im wesentlichen den zu Figur 14 beschriebenen, mit dem Unterschied, dass hier die erfindungsgemäßen Küvetten wie die Alu-Küvetten einen Elektrodenabstand von 2 mm aufweisen. Die hier verwendeten Küvetten weisen ferner jeweils Elektroden aus mit ca. 33 - 37 Gew.-% Kohlefasern und ca. 23 - 27 Gew.-% Graphit dotiertem Polyamid (PA6 oder PA66) auf (Konzentration der Dotierung insgesamt: ca. 55 - 65 Gew.-%). Zur Kompensation des höheren Widerstands der Kunststoffelektroden wurden hier unterschiedliche Pulsparameter verwendet (Kunststoff: 1000 V, 100 µs und U_{Anfang} = 102 V, 75 mC und Aluminium: 800 V, 100 µs und U_{Anfang} = 90 V, 75 mC). Auch hier konnten durch die Verwendung der erfindungsgemäßen Kunststoffelektroden die Transfektionseffizienzen leicht gesteigert und die Mortalitätsraten leicht verringert werden.

### Liste der verwendeten Abkürzungen:

Außer den im Duden gebräuchlichen, wurden folgende Abkürzungen verwendet:
- A: Ampere
- C: Coulomb
- CHO: chinese hamster ovary
- cm: Zentimeter
- DNA: Desoxyribonukleinsäure
- Gew.-%: Gewichtsprozent
- h: Stunden
- HL-60: human lymphoma 60
- HUVEC: Humane Nabelschnurvenen-Endothelzellen
- kV: Kilovolt
- mC: Millicoulomb
- mM: Millimolar
- ms: Millisekunden
- PA: Polyamid
- PBMC: peripheral blood mononuclear cells
- PBS: Phosphat buffered Saline
- pH: negativer dekadischer Logarithmus der Wasserstoffionen-Konzentration
- PJ: Propidiumjodid
- RNA: Ribonukleinsäure
- RPMI: Rosewell Park Memorial Institute
- µg: Mikrogramm
- µl: Mikroliter
- µs: Mikrosekunden
- U: Spannung
- U_{Anfang}: Anfangsspannung
- V: Volt

### Bezugszeichenliste:

- 1: Versuchsanordnung
- 2: Abstandhalterplatte
- 3: Elektrode
- 4: Elektrode
- 5: Ausschnitt
- 6: Kupferplatte
- 7: Kupferplatte
- 8: Gewindeabschnitt
- 9: Gewindeabschnitt
- 10: Innenraum
- 11: Innenkanten
- 12: Versuchsanordnung
- 13: Kupferdraht
- 14: Kupferdraht
- 20: Behälter
- 21: äußere Begrenzung
- 22: Innenraum
- 23: Seitenwand
- 24: Seitenwand
- 25: Elektrode
- 26: Elektrode
- 27: Spritzkanal
- 28: Spritzkanal
- 30: Behälter
- 31: äußere Begrenzung
- 32: Innenraum
- 33: Elektrode
- 34: Elektrode
- 35: Seitenwand
- 36: Seitenwand
- 37: Spritzkanal
- 38: Spritzkanal
- 39: Abschrägung

## Patentansprüche

1. Behälter (20, 30) zur Aufnahme einer wässrigen Lösung, und insbesondere von Zellen, Zellderivaten, subzellulären Partikeln und/oder Vesikeln, welcher zumindest teilweise von einer äußeren Begrenzung (21) gebildet ist, die einen Innenraum (22, 32) zur Aufnahme der Lösung bildet, und welcher mindestens einen Bereich aufweist, der beim Anlegen einer elektrischen Spannung und einer anschließenden Entladung als Elektrode (25, 26, 33, 34) dient, **dadurch gekennzeichnet, dass** mindestens eine Elektrode (25, 26, 33, 34) aus einem leitfähigen Kunststoffmaterial besteht, das zumindest auf einem Kunststoff basiert, der mit mindestens einem leitfähigen Stoff dotiert ist, wobei die Dotierung in dem Kunststoff in einer Konzentration von insgesamt 20 - 80 Gew.-% vorliegt.

2. Behälter nach Anspruch 1, **dadurch gekennzeichnet, dass** die Dotierung aus Kohlefasern, Graphit, Ruß und/oder Kohlenstoffnanotubes besteht.

3. Behälter nach Anspruch 1 oder 2, **dadurch gekennzeichnet, dass** die Dotierung in dem Kunststoff in einer Konzentration von insgesamt 20 - 60 Gew.-%, vorzugsweise 40 - 60 Gew.-%, weiter bevorzugt 50 - 60 Gew.-%, insbesondere 55 - 60 Gew.-%, vorliegt.

4. Behälter nach Anspruch 1 oder 2, **dadurch gekennzeichnet, dass** die Dotierung in dem Kunststoff in einer Konzentration von insgesamt 40 - 80 Gew.-%, vorzugsweise 50 - 80 Gew.-%, weiter bevorzugt 60 - 80 Gew.-%, besonders bevorzugt 70 - 80 Gew.-%, insbesondere 74 - 76 Gew.-% vorliegt.

5. Behälter nach einem der Ansprüche 1 bis 4, **dadurch gekennzeichnet, dass** der Kunststoff Polycarbonat, Polyetheretherketon, Polypropylen, Polyamid, Polyphenylensulfid oder ein Gemisch dieser Polymere ist oder zumindest auf einem oder mehreren dieser Polymere basiert und/oder dass der Kunststoff ein intrinsisch leitender Kunststoff ist.

6. Behälter nach Anspruch 5, **dadurch gekennzeichnet, dass** der intrinsisch leitende Kunststoff ein Polyanilin, Polyacetylen, Poly-para-phenylen, Poly-para-phenylensulfid, Polypyrrol, Polythiophen, Polypropylen oder ähnliches ist oder zumindest auf einem oder mehreren dieser Polymere basiert.

7. Behälter nach einem der Ansprüche 1 bis 6, **dadurch gekennzeichnet, dass** die äußere Begrenzung (21, 31) aus Kunststoff, bevorzugt transparentem Kunststoff, gebildet ist.

8. Behälter nach Anspruch 7, **dadurch gekennzeichnet, dass** die äußere Begrenzung (21, 31) aus dem gleichen Kunststoff besteht, auf dem auch die zumindest eine Elektrode (25, 26, 33, 34) basiert.

9. Behälter nach einem der Ansprüche 1 bis 8, **dadurch gekennzeichnet, dass** die zumindest eine Elektrode (25, 26, 33, 34) in die äußere Begrenzung (21, 31) integriert ist.

10. Behälter nach einem der Ansprüche 1 bis 9, **dadurch gekennzeichnet, dass** dieser zumindest zwei Elektroden (25, 26, 33, 34) aufweist, die aus dem gleichen Material bestehen.

11. Behälter nach einem der Ansprüche 1 bis 10, **dadurch gekennzeichnet, dass** zumindest zwei Elektroden (25, 26, 33, 34) aus unterschiedlichen Materialien bestehen.

12. Behälter nach einem der Ansprüche 1 bis 11, **dadurch gekennzeichnet, dass** zumindest eine Elektrode (25, 26, 33, 34) aus mit 25 - 45 Gew.-%, vorzugsweise 30 - 40 Gew.-%, insbesondere 33 - 37 Gew.-%, Kohlefasern und 15 - 35 Gew.-%, vorzugsweise 20 - 30 Gew.-%, insbesondere 23 - 27 Gew.-%, Graphit dotiertem Polyamid, insbesondere Polyamid 66 oder Polyamid 6, besteht.

13. Behälter nach einem der Ansprüche 1 bis 11, **dadurch gekennzeichnet, dass** zumindest eine Elektrode (25, 26, 33, 34) aus mit 30 - 50 Gew.-%, vorzugsweise 35 - 45 Gew.-%, insbesondere 39 - 41 Gew.-%, Kohlefasern und 25 - 45 Gew.%, vorzugsweise 30 - 40 Gew.-%, insbesondere 34 - 36 Gew.-%, Graphit dotiertem Polyamid, insbesondere Polyamid 66 oder Polyamid 6, besteht.

14. Behälter nach einem der Ansprüche 1 bis 11, **dadurch gekennzeichnet, dass** zumindest eine Elektrode (25, 26, 33, 34) aus mit 15 - 40 Gew.-%, vorzugsweise 20 Gew.%, Kohlefasern und 1 - 40 Gew.-%, vorzugsweise 15 Gew.-%, Graphit dotiertem Polycarbonat besteht.

15. Behälter nach einem der Ansprüche 1 bis 11, **dadurch gekennzeichnet, dass** zumindest eine Elektrode (25, 26, 33, 34) aus mit 30 - 50 Gew.-%, vorzugsweise 40 Gew.-%, Kohlefasern dotiertem Polyetheretherketon besteht.

16. Behälter nach einem der Ansprüche 1 bis 11, **dadurch gekennzeichnet, dass** zumindest eine Elektrode (25, 26, 33, 34) aus mit 20 - 40 Gew.-%, vorzugsweise 30 Gew.-%, Kohlefasern dotiertem Polyamid, vorzugsweise Polyamid 66, besteht.

17. Behälter nach einem der Ansprüche 1 bis 11, **dadurch gekennzeichnet, dass** zumindest eine Elektrode (25, 26, 33, 34) aus mit 20 Gew.-% Kohlefasern dotiertem Polypropylen besteht.

18. Behälter nach einem der Ansprüche 1 bis 11, **dadurch gekennzeichnet, dass** zumindest eine Elektrode (25, 26, 33, 34) aus mit 30 - 50 Gew.-%, vorzugsweise 40 Gew.-%, Kohlefasern dotiertem Polyphenylensulfid besteht.

19. Behälter nach einem der Ansprüche 1 bis 18, **dadurch gekennzeichnet, dass** die äußere Begrenzung (21, 31) zumindest eine Öffnung zum Zuleiten der Lösung und zumindest eine Öffnung zum Ableiten der Lösung aufweist.

20. Behälteranordnung bestehend aus wenigstens zwei, vorzugsweise 6, 12, 24, 48, 96 oder mehr, Behältern (20, 30) nach einem der Ansprüche 1 bis 18, die zu einer Einheit verbunden sind.

21. Verfahren zur Herstellung der Behälter oder Behälteranordnung nach einem der Ansprüche 1 bis 20, **dadurch gekennzeichnet, dass** der Behälter (20, 30) oder die Behälteranordnung im 2-Komponenten-Spritzguss hergestellt wird, wobei zunächst die äußere Begrenzung (21, 31) mit mindestens einem ausgesparten Fenster gespritzt wird und anschließend zumindest in das mindestens eine Fenster das leitfähigen Kunststoffmaterial aus dotiertem Kunststoff eingespritzt wird, oder wobei zunächst die mindestens eine Elektrode (25, 26, 33, 34) aus dem dotierten Kunststoff gespritzt wird und anschließend um die mindestens eine Elektrode (25, 26, 33, 34) herum die äußere Begrenzung (21, 31) gespritzt wird.

22. Verfahren zur Behandlung von Zellen, Zellderivaten, subzellulären Partikeln und/oder Vesikeln mit elektrischem Strom, insbesondere zur Elektroporation oder Elektrofusion, umfassend:
a) Überführen der Zellen, Zellderivate, subzellulären Partikel und/oder Vesikel in den Innenraum zumindest eines Behälters (20, 30) nach einem der Ansprüche 1 bis 19 oder zumindest eines Behälters einer Behälteranordnung nach Anspruch 20, wobei der Behälter (20, 30) mindestens eine Elektrode (25, 26, 33, 34) aus dem dotierten Kunststoff aufweist, sowie zumindest eine weitere Elektrode (25, 26, 33, 34) vorgesehen ist, und
b) Anlegen einer elektrischen Spannung an die Elektroden (25, 26, 33, 34) und Erzeugung eines Stromflusses im Innenraum (22, 32) des Behälters (20, 30).

23. Verfahren nach Anspruch 22, **dadurch gekennzeichnet, dass** der elektrische Strom eine Stromdichte von bis zu 120 A/cm², vorzugsweise 80 A/cm², erreicht.

24. Verfahren nach Anspruch 22 oder 23, wobei biologisch aktive Moleküle, insbesondere Nukleinsäuren, in der Lösung gelöst sind und durch einen Spannungspuls mit einer Feldstärke von 2 bis 10 kV*cm⁻¹ und einer Dauer von 10 bis 200 µs ein Einbringen dieser biologisch aktiven Moleküle in lebende Zellen erreicht wird.

25. Verfahren nach Anspruch 24, wobei das Einbringen der biologisch aktiven Moleküle in die Zellen durch einen auf den Spannungspuls ohne Unterbrechung folgenden Stromfluss mit einer Stromdichte von 2 bis 14 A/cm², vorzugsweise 5 A/cm², und einer Dauer von 1 bis 100 ms, vorzugsweise 50 ms, erreicht wird.

## Claims

1. Container (20, 30) for receiving an aqueous solution, and in particular cells, derivatives of cells, subcellular particles and/or vesicles, which is formed at least partially by an outer limit (21) which forms an inner chamber (22, 32) for receiving said solution, and which comprises at least one area which acts as an electrode (25, 26, 33, 34) when an electric voltage is applied and a subsequent discharge occurs, wherein said at least one electrode (25, 26, 33, 34) is made of a conductive synthetic material which is, or is at least based on, a plastic material which is doped with at least one conductive substance, and wherein the overall concentration of said dope in said plastic material is 20 - 80 % w/w.

2. Container according to claim 1, wherein said dope consists of carbon fibers, graphite, soot and/or carbon nanotubes.

3. Container according to claim 1 or 2, wherein the overall concentration of said dope in said plastic material is 20 - 60 % w/w, preferably 40 - 60 % w/w, most preferred 50 - 60 % w/w, in particular 55 - 60 % w/w.

4. Container according to claim 1 or 2, wherein the overall concentration of said dope in said plastic material is 40 - 80 % w/w, preferably 50 - 80 % w/w, more preferred 60 - 80 % w/w, most preferred 70 - 80 % w/w, in particular 74 - 76 % w/w.

5. Container according to any one of the claims 1 to 4, wherein said plastic material is polycarbonate, polyetheretherketone, polypropylene, polyamide, polyphenylensulfide or a mixture of these polymers, or at least based on one or several of these polymers, and/or wherein said plastic material is an intrinsically conductive synthetic material.

6. Container according claim 5, wherein said intrinsically conductive synthetic material is polyaniline, polyacetylene, poly-para-phenylene, poly-para-phenylensulfide, polypyrroles, polythiophene, polypropylene or the like, or at least based on one or several of these polymers.

7. Container according to any one of the claims 1 to 6, wherein said outer limit (21, 31) is made of synthetic material, preferably transparent plastic material.

8. Container according to claim 7, wherein said outer limit (21, 31) is made of the same plastic material as the plastic material on which said at least one electrode (25, 26, 33, 34) is based.

9. Container according to any one of the claims 1 to 8, wherein said at least one electrode (25, 26, 33, 34) is integrated into said outer limit (21, 31).

10. Container according to any one of the claims 1 to 9 comprising at least two electrodes (25, 26, 33, 34) being made of the same material.

11. Container according to any one of the claims 1 to 10, wherein said at least two electrodes (25, 26, 33, 34) are made of different materials.

12. Container according to any one of the claims 1 to 11, wherein said at least one electrode (25, 26, 33, 34) is made of polyamide, in particular polyamide 66 or polyamide 6, doped with 25 - 45 % w/w, preferably 30 - 40 % w/w, in particular 33 - 37 % w/w, carbon fibers and 15 - 35 % w/w, preferably 20 - 30 % w/w, in particular 23 - 27 % w/w, graphite.

13. Container according to any one of the claims 1 to 11, wherein said at least one electrode (25, 26, 33, 34) is made of polyamide, in particular polyamide 66 or polyamide 6, doped with 30 - 50 % w/w, preferably 35 - 45 % w/w, in particular 39 - 41 % w/w, carbon fibers and 25 - 45 % w/w, preferably 30 - 40 % w/w, in particular 34 - 36 % w/w, graphite.

14. Container according to any one of the claims 1 to 11, wherein said at least one electrode (25, 26, 33, 34) is made of polycarbonate doped with 15 - 40 % w/w carbon fibers, preferably 20 % w/w carbon fibers, and 1 - 40 % w/w graphite, preferably 15 % w/w graphite.

15. Container according to any one of the claims 1 to 11, wherein said at least one electrode (25, 26, 33, 34) is made of polyetheretherketone doped with 30 - 50 % w/w carbon fibers, preferably 40 % w/w carbon fibers.

16. Container according to any one of the claims 1 to 11, wherein said at least one electrode (25, 26, 33, 34) is made of polyamide, preferably polyamide 66, doped with 20 - 40 % w/w carbon fibers, preferably 30 % w/w carbon fibers.

17. Container according to any one of the claims 1 to 11, wherein said at least one electrode (25, 26, 33, 34) is made of polypropylene doped with 20 % w/w carbon fibers.

18. Container according to any one of the claims 1 to 11, wherein said at least one electrode (25, 26, 33, 34) is made of polyphenylensulfide doped with 30 - 50 % w/w carbon fibers, preferably 40 % w/w carbon fibers.

19. Container according to any one of the claims 1 to 18, wherein said outer limit (21, 31) comprises at least one opening for supplying said solution and at least one opening for draining off said solution.

20. Container arrangement comprising at least two, preferably 6, 12, 24, 48, 96 or more, containers (20, 30) according to any one of the claims 1 to 18 being joined to build one unit.

21. Method for producting containers or container arrangements according to any one of the claims 1 to 20, wherein said container (20, 30) or said container arrangement is produced by two-component injection moulding, wherein at first the outer limit (21, 31) is injection-moulded leaving one recessed window and the conductive synthetic material being made of doped plastic is subsequently injection-moulded into said at least one window, or alternatively, wherein at first said at least one electrode (25, 26, 33, 34) is injection-moulded of said doped plastic material and said outer limit (21, 31) is subsequently injection-moulded around said at least one electrode (25, 26, 33, 34).

22. Method for treatment of cells, derivatives of cells, subcellular particles and/or vesicles by means of electric current, in particular for electroporation or electrofusion, comprising:
a) Transferring said cells, derivatives of cells, subcellular particles and/or vesicles into an inner chamber (22, 32) of at least one container (20, 30) according to any one of the claims 1 to 19 or at least one container of a container arrangement according to claim 20, wherein said container (20, 30) comprises at least one electrode (25, 26, 33, 34) being made of a doped synthetic material, and at least one further electrode (25, 26, 33, 34), and
b) Applying voltage to said electrodes (25, 26, 33, 34) and generating a current flow in said inner chamber (22, 32) of said container (20, 30).

23. Method according to claim 22, wherein said electric current reaches a current density up to 120 A/cm², preferably 80 A/cm².

24. Method according to claim 22 or 23, wherein biologically active molecules, in particular nucleic acids, are solved in said solution, and transfer of said biologically active molecules into living cells is achieved via a voltage pulse having a field strength of 2 to 10 kV*cm⁻¹ and a duration of 10 to 200 µs.

25. Method according to claim 24, wherein said transfer of said biologically active molecules into said cells is achieved by a current flow following said voltage pulse without interruption, having a current density of 2 to 14 A*cm⁻², preferably 5 A*cm⁻², and a duration of 1 to 100 ms, preferably 50 ms.

## Revendications

1. Réceptacle (20, 30) pour recueillir une solution aqueuse, et plus particulièrement des cellules, des dérivés cellulaires, des particules subcellulaires et/ou des vésicules, qui est constitué au moins partiellement d'une enveloppe externe de délimitation (21) formant un espace intérieur (22, 32) pour recueillir la solution et qui présente au moins une zone qui sert d'électrode (25, 26, 33, 34) lors de l'application d'une tension électrique et d'une décharge associée, **caractérisé en ce qu'**au moins une électrode (25, 26, 33, 34) se compose d'un matériau plastique conducteur basé au moins sur un plastique dopé avec au moins une matière conductrice, la dotation en matière conductrice dopante étant présente dans le plastique en une concentration totale de 20 à 80 % en poids.

2. Réceptacle selon la revendication 1, **caractérisé en ce que** la dotation en matière conductrice dopante se compose de fibres de carbone, de graphite, de noir de fumée et/ou de nanotubes de carbone.

3. Réceptacle selon la revendication 1 ou 2, **caractérisé en ce que** la dotation en matière conductrice dopante dans le plastique est présente en une concentration totale de 20 à 60 en % en poids, de préférence de 40 à 60 % en poids, selon une préférence particulière de 50 à 60 % en poids, plus particulièrement de 55 à 60 % en poids.

4. Réceptacle selon la revendication 1 ou 2, **caractérisé en ce que** la dotation en matière conductrice dopante dans le plastique est présente en une concentration totale de 40 à 80 % en poids, de préférence de 50 à 80 % en poids, selon une préférence particulière de 60 à 80 % en poids, selon une préférence toute particulière de 70 à 80 % en poids, plus particulièrement de 74 à 76 % en poids.

5. Réceptacle selon l'une des revendications 1 à 4, **caractérisé en ce que** le plastique est du polycarbonate, du polyétheréthercétone, du polypropylène, du polyamide, du polyphénylènesulfone ou un mélange de ces polymères ou est basé sur au moins un ou plusieurs de ces polymères et/ou **en ce que** le plastique est un plastique à conductibilité intrinsèque.

6. Réceptacle selon la revendication 5, **caractérisé en ce que** le plastique à conductibilité intrinsèque est de la polyaniline, du polyacétylène, du poly-paraphénylène, du poly-paraphénylènesulfone, du polypyrrol, du polythiophène, du polypropylène ou analogues ou est basé sur au moins un ou plusieurs de ces polymères.

7. Réceptacle selon l'une des revendications 1 à 6, **caractérisé en ce que** l'enveloppe externe de délimitation (21, 31) est constituée de plastique, de préférence de plastique transparent.

8. Réceptacle selon la revendication 7, **caractérisé en ce que** l'enveloppe externe de délimitation (21, 31) se compose du même plastique que celui sur lequel l'une au moins des électrodes (25, 26, 33, 34) est aussi basée.

9. Réceptacle selon l'une des revendications 1 à 8, **caractérisé en ce que** l'une au moins des électrodes (25, 26, 33, 34) est intégrée dans l'enveloppe externe de délimitation (21, 31).

10. Réceptacle selon l'une des revendications 1 à 9, **caractérisé en ce que** celui-ci présente au moins deux électrodes (25, 26, 33, 34) qui sont composées du même matériau.

11. Réceptacle selon l'une des revendications 1 à 10, **caractérisé en ce qu'**au moins deux électrodes (25, 26, 33, 34) sont composées de matériaux différents.

12. Réceptacle selon l'une des revendications 1 à 11, **caractérisé en ce qu'**au moins une électrode (25, 26, 33, 34) se compose de 25 à 45 % en poids, de préférence de 30 à 40 % en poids, plus particulièrement de 33 à 37 % en poids de fibres de carbone et de 15 à 35 % en poids, de préférence de 20 à 30 % en poids, plus particulièrement de 23 à 27 % en poids de polyamide dopé avec du graphite, plus particulièrement de polyamide 66 ou de polyamide 6.

13. Réceptacle selon l'une des revendications 1 à 11, **caractérisé en ce qu'**au moins une électrode (25, 26, 33, 34) se compose de 30 à 50 % en poids, de préférence de 35 à 45 % en poids, plus particulièrement de 39 à 41 % en poids de fibres de carbone et de 25 à 45 % en poids, de préférence de 30 à 40 % en poids, plus particulièrement de 34 à 36 % en poids de polyamide dopé avec du graphite, plus particulièrement de polyamide 66 ou de polyamide 6.

14. Réceptacle selon l'une des revendications 1 à 11, **caractérisé en ce qu'**au moins une électrode (25, 26, 33, 34) se compose de 15 à 40 % en poids, de préférence de 20 % en poids de fibres de carbone et de 1 à 40 % en poids, de préférence de 15 % en poids, de polycarbonate dopé avec du graphite.

15. Réceptacle selon l'une des revendications 1 à 11, **caractérisé en ce qu'**au moins une électrode (25, 26, 33, 34) se compose de 30 à 50 % en poids, de préférence de 40 % en poids de polyétheréthercétone dopé avec des fibres de carbone.

16. Réceptacle selon l'une des revendications 1 à 11, **caractérisé en ce qu'**au moins une électrode (25, 26, 33, 34) se compose de 20 à 40 % en poids, de préférence de 30 % en poids de polyamide dopé avec des fibres de carbone, de préférence de polyamide 66.

17. Réceptacle selon l'une des revendications 1 à 11, **caractérisé en ce qu'**au moins une électrode (25, 26, 33, 34) se compose de 20 % en poids de polypropylène dopé avec des fibres de carbone.

18. Réceptacle selon l'une des revendications 1 à 11, **caractérisé en ce qu'**au moins une électrode (25, 26, 33, 34) se compose de 30 à 50 % en poids, de préférence de 40 % en poids de polyphénylènesulfone dopé avec des fibres de carbone.

19. Réceptacle selon l'une des revendications 1 à 18, **caractérisé en ce que** l'enveloppe externe de délimitation (21, 31) présente au moins une ouverture pour l'admission de la solution et au moins une ouverture pour l'évacuation de la solution.

20. Arrangement de réceptacles composé d'au moins deux, de préférence de 6, 12, 24, 48, 96 ou plus, réceptacles (20, 30) selon l'une des revendications 1 à 18, qui sont reliés en une unité.

21. Procédé pour la fabrication des réceptacles ou de l'arrangement de réceptacles selon l'une des revendications 1 à 20, **caractérisé en ce que** le réceptacle (20, 30) ou l'arrangement de réceptacles est fabriqué par moulage par injection à 2 composants, en réalisant tout d'abord l'injection de l'enveloppe externe de délimitation (21, 31) avec au moins une épargne formant fenêtre et en injectant ensuite dans l'une au moins des fenêtres au moins le matériau plastique conducteur en plastique dopé, ou en réalisant tout d'abord la fabrication, par injection à partir du plastique dopé, de l'une au moins des électrodes (25, 26, 33, 34) et en réalisant ensuite autour de l'une au moins des électrodes (25, 26, 33, 34) l'injection de l'enveloppe externe de délimitation (21, 31).

22. Procédé de traitement de cellules, de dérivés cellulaires, de particules subcellulaires et/ou de vésicules par courant électrique, plus particulièrement en vue de l'électroporation ou de l'électrofusion, comportant :
a) le transfert des cellules, dérivés cellulaires, particules subcellulaires et/ou vésicules dans l'espace intérieur d'au moins un réceptacle (20, 30) selon l'une des revendications 1 à 19 ou d'au moins un réceptacle d'un arrangement de réceptacles selon la revendication 20, le réceptacle (20, 30) présentant au moins une électrode (25, 26, 33, 34) composée du plastique dopé et au moins une autre électrode (25, 26, 33, 34) étant de même prévue, et
b) l'application d'une tension électrique aux électrodes (25, 26, 33, 34) et la création d'un flux de courant dans l'espace intérieur (22, 32) du réceptacle (20, 30).

23. Procédé selon la revendication 22, **caractérisé en ce que** le courant électrique possède une densité de courant allant jusqu'à 120 A/cm², de préférence de 80 A/cm².

24. Procédé selon la revendication 22 ou 23, dans lequel des molécules biologiquement actives, plus particulièrement des acides nucléiques, sont mises en solution et dans lequel par une pulsation de courant ayant une intensité de champ de 2 à 10 kV*cm⁻¹ et une durée de 10 à 200 µs, on parvient à introduire ces molécules biologiquement actives dans des cellules vivantes.

25. Procédé selon la revendication 24, dans lequel on parvient à introduire dans les cellules les molécules biologiquement actives par un flux de courant succédant sans interruption à la pulsation de courant et ayant une densité de courant de 2 à 14 A/cm², de préférence de 5 A/cm², et une durée de 1 à 100 ms, de préférence de 50 ms.
